# EUROPÄISCHE PATENTSCHRIFT

(11) **EP 2 088 869 B1**
(45) Veröffentlichungstag und Bekanntmachung des Hinweises auf die Patenterteilung: **19.05.2010**
(21) Anmeldenummer: 06806087.0
(22) Anmeldetag: 06.10.2006
(51) Int. Cl.: A22C 25/00, G01N 33/12

(54) **VERFAHREN UND VORRICHTUNG ZUM BEARBEITEN VON IN MEHRZAHL ENTLANG EINER BEARBEITUNGSLINIE GEFÖRDERTEN FISCH-, GEFLÜGEL- ODER ANDERE FLEISCHPRODUKTEN**
METHOD AND DEVICE FOR PROCESSING FISH, POULTRY, OR OTHER MEAT PRODUCTS TRANSPORTED IN MULTITUDE ALONG A PROCESSING LINE
PROCÉDÉ ET DISPOSITIF POUR TRAITER DES PRODUITS À BASE DE POISSON, VOLAILLE, OU D'AUTRES PRODUITS CARNÉS TRANSPORTÉS EN QUANTITÉS LE LONG D'UNE CHAÎNE DE TRAITEMENT

(43) Veröffentlichungstag der Anmeldung: 19.08.2009
(73) Patentinhaber: Nordischer Maschinenbau Rud. Baader GmbH + Co. KG, 23560 Lübeck (DE)
(72) Erfinder: HAUCKE, Hartmut A., 23560 Lübeck (DE); MILLER, Ralph Anderson, 23560 Lübeck (DE); HEARN, Paul M., St. John's NF A1E 1C1 (CA); NICHOLAS, Shawn, Kansas City, KS 66115 (US)
(74) Vertreter: Wenzel & Kalkoff
(86) Internationale Anmeldenummer: PCT/EP2006/009682
(87) Internationale Veröffentlichungsnummer: WO 2008/043370

(56) Entgegenhaltungen:
- EP-A1- 0 128 889
- DE-C1- 3 524 906
- US-A1- 5 847 382
- US-A1- 2003 098 409
- US-B1- 6 532 064

## Beschreibung

Die Erfindung betrifft ein Verfahren und eine Vorrichtung zum Bearbeiten von in Mehrzahl entlang einer Bearbeitungslinie geförderten Fisch-, Geflügel- oder anderen Fleischprodukten. Es handelt sich um Fleischprodukte, die nicht verzehrbare oder vom Verzehr auszuschließende abzutrennende Bestandteile (Abtrennbestandteile) aufweisen. Es ist bekannt, dass durch das Abtrennen gewonnene Fleischprodukte, nämlich Filets mittels einer automatischen Inspektionseinrichtung auf zurückgebliebene Fehlstellen, nämlich Knochenreste in Fischfilets oder Geflügel-Filetstücken geprüft werden.

Zum Beispiel ist es aus EP-A-0 128 889 bekannt, dass Fischfilets, aus denen Pinbones herausgetrennt worden sind, mit einer Sensoreinrichtung inspiziert werden, um zurückgebliebene Pinbonereste festzustellen. Pinbonereste aufweisende Fischfilets werden einer Inspektionsstation zugeführt, in der der Knochenrest auf einem Display angezeigt wird, um durch Bearbeitungspersonal manuell entfernt zu werden. Die Inspektionsergebnisse werden auch genutzt, um Informationen zur Produktqualität zu erhalten.

Beim Filetieren von Fisch- und Fleischprodukten erhält man eine höhere Fleischausbeute dadurch, dass Schnitte näher am Knochen ausgeführt werden. Damit ist jedoch das unbestimmte Risiko verbunden, dass gefährliche verborgene Knochenreste, zum Beispiel des Gabelbeins bei Geflügel oder des Schlüsselbeins bei Fisch bzw. damit verunreinigte Bereiche unkontrolliert in dem Filet (Verzehrprodukt) zurückbleiben. Diese Situation tritt zum Beispiel verschärft auf, wenn beim Schlachten Knochensplitter und/- oder Blutergüsse entstehen. Um das Knochenrisiko zu reduzieren, sind Verarbeiter daher bestrebt, ihre manuellen oder automatisierten Filetiermaschinen so einzustellen, dass möglichst der gesamte Knochen-/Verunreinigungsbereich abgetrennt wird. Dadurch wird die Ausbeute zur Filetgewinnung beeinträchtigt, da in unerwünschtem Maß auch defektfreies Fleisch weggeschnitten wird.

Zum Entbeinen bzw. Filetieren von Geflügel-Vorderhälften sind Maschinen bekannt, die die Geflügelbrust unter Verwendung computergesteuerter Schneidmesser und Schabeklingen abtrennen. Die abgetrennten Geflügelbruststücke (Verzehrprodukte) werden mittels Förderband zu einer Station gebracht, an der Arbeiter die Filets manuell auf Knochenreste prüfen und diese ebenfalls manuell abtrennen. Es ist üblich, dann die als defektfrei festgestellten und getrennten Filets einer Endinspektion zu unterziehen, die manuell oder mit Hilfe einer Röntgeneinrichtung durchgeführt wird.

Um Filets praktisch restlos zu entgräten und von Knochenbestandteilen zu befreien, wird es gerade auch in Verbindung mit einer automatischen Inspektion hinsichtlich höchster Sicherheit zum Entfernen von Defektresten zweckmäßig sein, im Anschluss an die automatische Inspektion durch Personal ein manuelles Trimmen durchzuführen. Selbstverständlich ist es von Bedeutung, dass die Tätigkeit des Trimmens möglichst einfach und zuverlässig gestaltet werden kann, um sie zu erleichtern und sicherer zu machen und gegebenenfalls auch Anforderungen an Personalqualifikation zu reduzieren. Zu diesem Zweck sind Hilfseinrichtungen bekannt, die mit Knochenresten behaftete Filets auf einem Monitor zeigen, um das Trimmen zu erleichtern. Selbst solche Monitor-Hilfssysteme können zu Personalermüdung oder -unaufmerksamkeit führen, wenn Defektreste in relativ großen Abständen, besonders unregelmäßig und/oder mit erheblicher Streuung in der Erscheinungsform auftreten.

Nach alledem bestehen Ziele der Erfindung darin, ein Verfahren und eine dafür eingerichtete Vorrichtung anzugeben, um bei Verzehrprodukten mit hoher Effektivität und höchster Sicherheit beim Abtrennen, insbesondere beim Filetieren anfallende Reste zu erfassen und zu beseitigen. Soweit im Gesamtprozess manuelle Bearbeitungen und/oder -kontrollen durchgeführt werden, sollen diese Tätigkeiten hinsichtlich auch der Personalwahl bzw. des Personaleinsatzes vereinfacht sein, und es sollen automatische und gesteuerte Bearbeitungen durchgeführt und verbessert werden können. Das Verfahren mit geeigneter Vorrichtung soll auch ein breites Anwendungsspektrum eröffnen, nämlich für jede Art und Form von Fisch-, Geflügel- oder anderen Fleischprodukten verwendbar sein. Das heißt, dass insbesondere ein Verfahren erreicht werden soll, das durchgeführt werden kann einerseits für abzutrennende Bestandteile sehr unterschiedlicher Beschaffenheit, Struktur und Zusammensetzung (Textur) sowie andererseits für Fleischprodukte in roher, halbfertiger oder fertiger Art und Form, also zum Beispiel auch für gebratenes oder sonstwie zum Verzehr zubereitetes Fleisch oder geformte Fleischmasse.

Die Ziele der Erfindung werden durch ein besonderes Verfahren und eine dazu gestaltete Vorrichtung erreicht. So ist die Erfindung durch ein Verfahren zum Bearbeiten von in Mehrzahl entlang einer Bearbeitungslinie geförderten Fisch-, Geflügel- oder anderen Fleischprodukten jeder Art und Form bestimmt, die nicht verzehrbare oder vom Verzehr auszuschließende abzutrennende Bestandteile (Abtrennbestandteile) wenigstens einer Art enthalten, wobei die Abtrennbestandteile von den Fleischprodukten getrennt werden und dadurch zum Verzehr gewonnene Verzehrprodukte mittels einer automatischen Inspektionseinrichtung auf beim Trennen zurückgebliebene Reste geprüft werden, umfassend folgende Schritte: i) Die Abtrennbestandteile werden von den Fleischprodukten derart getrennt, dass bei einer Anzahl der Verzehrprodukte minimierte Reste wenigstens einer Art zum weiteren Bearbeiten der Verzehrprodukte, insbesondere zum Optimieren der Verzehrproduktgewinnung zugelassen und herbeigeführt werden; ii) die automatische Inspektionseinrichtung wird zum Feststellen der minimierten Toleranzreste eingestellt und iii) von der Inspektionseinrichtung abgegebene und mit den Toleranzresten behaftete und restfreie Verzehrprodukte werden separiert.

Eine für das erfindungsgemäße Verfahren vorgesehene erfindungsgemäße Vorrichtung umfasst eine Trenneinrichtung zum Abtrennen von nicht verzehrbaren oder vom Verzehr auszuschließenden Bestandteilen (Abtrennbestandteile), eine der Trenneinrichtung nachgeordnete automatische Inspektionseinrichtung zum Prüfen der durch die Abtrennung gewonnenen Verzehrprodukte auf zurückgebliebene Reste und ein der Inspektionseinrichtung nachgeordnetes Mittel zum Separieren restbehafteter und restfreier Verzehrprodukte, wobei die Trenneinrichtung mit wenigstens einem Abtrennwerkzeug derart eingerichtet ist, dass bei einer Anzahl der Verzehrprodukte minimierte Toleranzreste wenigstens einer Art zum weiteren Bearbeiten der Verzehrprodukte, insbesondere zum Optimieren der Verzehrproduktgewinnung zurückbleiben, dass die automatische Inspektionseinrichtung zum Feststellen der minimierten Toleranzreste eingerichtet und mit einer Separatoreinrichtung verbunden ist, die restfreie und restbehaftete Verzehrprodukte nach Maßgabe der Inspektionsergebnisse der Inspektionseinrichtung unterscheidet und voneinander getrennt abgibt.

Der Erfindung ist es eigen, zum Abtrennen der Abtrennbestandteile bei einer Anzahl der Verzehrprodukte ganz gezielt ein Minimum reduzierte Reste zu bewirken. Das heißt, dass Reste in einem bestimmten Maß sowie für eine Anzahl in Mehrzahl geförderter gleichartiger Verzehrprodukte gezielt toleriert werden. Demgemäß ist die Angabe "Toleranzreste" zu verstehen. Im wesentlichen Unterschied zu üblichen Einstellungen für das Abtrennen von Gabelbeinen, Schlüsselbeinen, Pinbones, also von Defekt-Bestandteilen, die in Filetstücken nicht enthalten sein dürfen, wobei die bekannten Einstellungen darin bestehen, dass Restbestandteile von vornherein so weitgehend wie möglich vermieden werden, ist erfindungsgemäß gefunden worden, dass das definierte Tolerieren von Restbestandteilen eine Reihe von besonderen Vorteilen für die Bearbeitung insbesondere hinsichtlich Effektivität, sicherer Entfernung zurückgebliebener Reste (Produktsicherheit) sowie Produktgewinnung und -qualität liefert. Mit dem Zulassen der Toleranzreste gelingt es nämlich, ein kontrolliertes Rest- oder Knochenrisiko vorzugeben und einzurichten. Dadurch wird die zu messende und zu ermittelnde Rest- oder Defektstelle hinsichtlich der Messung und Ermittlung zur Inspektion besonders qualifiziert und quantifiziert. Es ist gefunden worden, dass sich die automatische Inspektionseinrichtung auf das Erfassen und Ermitteln der zugelassenen Reste (Toleranzreste) besonders zuverlässig und sicher einstellen lässt. Damit erreicht man, dass das Separieren der von der Inspektionseinrichtung abgegebenen und mit den Toleranzresten behafteten Verzehrprodukte von den restfreien Verzehrprodukten gleichfalls besonders sicher ist.

Die erreichte hohe Erfassungs-Sicherheit ist maßgeblich und wesentlich für das anschließende Separieren. Insbesondere entstehen Vorteile zum Abtrennen der Toleranzreste von den detektierten und separierten Verzehrprodukten. Aufgrund der tolerierten Reste treten diese bei der Mehrzahl entlang der Bearbeitungslinie zu bearbeitender, artgleicher und in der Form entsprechender Stücke mit einer Häufigkeit auf, die sich günstig auf die Trimmtätigkeit durch Personal auswirkt. Auch wird das Muster, nämlich das optische Auftreten der (zu beseitigenden) Toleranzreste in einem gewissen Maß vereinheitlicht oder stabilisiert, wodurch ebenfalls eine Begünstigung zur manuellen Bearbeitung erreicht wird. Dies führt auch dazu, dass die Produktlinie mit höherer Geschwindigkeit betrieben werden kann. Mit den erfindungsgemäßen Maßnahmen können insbesondere Fisch- und Geflügelprodukte im natürlichen Zustand besonders effektiv hinsichtlich der Produktgewinnung und Produktqualität sowie hinsichtlich eines optimalen Arbeitsablaufs bearbeitet werden. Doch lassen sich aufgrund der erfindungsgemäßen Maßnahme, dass nach dem Abtrennen gezielt minimierte Produktreste zurückgelassen werden, auch Anwendungen durchführen, bei denen die minimierten Reste ganz andere Texturen als Knochenreste aufweisen. Stets kommt es darauf an, dass eine bestimmte und damit qualifizierte und quantifizierte Textur zur Messung und Ermittlung mittels der Inspektionseinrichtung gezielt toleriert wird. Aufgrund der Schwankungsbreite des örtlichen Auftretens in der Folge der Mehrzahl geförderter gleichartiger Produkte macht man sich mit der Minimierung zu Nutze, dass ein Teil der Produkte restfrei ist, während ein anderer Teil der Produkte über den Arbeitsablauf verteilt die abzutrennenden Reste aufweist. Die Einstellung des Maßes der minimierten Reste kann zweckmäßig dazu genutzt werden, die Häufigkeit des Auftretens von Produkten mit tolerierten Resten hinsichtlich gewünschter Bearbeitung einzustellen. In jedem Fall erreicht man eine große Produktausbeute.

Eine vorteilhafte Ausgestaltung der Erfindung besteht darin, dass mittels der Inspektionseinrichtung wenigstens eine Toleranzreste repräsentierende Textur und gegebenenfalls wenigstens eine weitere Substanz-Textur der Verzehrprodukte gemessen werden und wenigstens eine Größe ermittelt wird, die eine qualitative, quantitative und/oder örtliche Textur-Substanzinformation der Verzehrprodukte repräsentiert und zum Beeinflussen wenigstens eines Abtrennprozesses vor und gegebenenfalls wenigstens eines weiteren Bearbeitungsprozesses vor und/oder hinter der Inspektionseinrichtung verwendet wird. Der erfindungsgemäßen Restminimierung kommt besondere Bedeutung zu, da damit - bei optimaler Gewinnung von restfreiem Fleisch - einerseits gezielt Verzehrprodukte zugelassen werden, die weitgehend definiert minimale Reste aufweisen, und andererseits solche, die frei von tolerierten Resten sind.

Für ein bestimmtes Produkt, zum Beispiel für in Mehrzahl geförderte zu bearbeitende Geflügel-Vorderhälften oder Geflügel-Brustkappen ist es zweckmäßig und wünschenswert, den minimierten Rest in Abhängigkeit von den tatsächlich auftretenden Verhältnissen einzustellen. Dies erreicht man gemäß einer erfindungsgemäßen Ausgestaltung dadurch, dass mittels der Inspektionseinrichtung wenigstens eine Toleranzreste repräsentierende Textur gemessen und wenigstens eine zugehörige Größe ermittelt wird, die eine qualitative, quantitative und/oder örtliche Textur-Substanzinformation der Verzehrprodukte repräsentiert und zum Beeinflussen wenigstens eines Abtrennprozesses vor der Inspektionseinrichtung verwendet wird. Um die gesamte Bearbeitung zu optimieren, kann die Inspektionseinrichtung im Rahmen des erfindungsgemäßen Verfahrens auch dazu genutzt werden, wenigstens eine weitere Substanztextur der Verzehrprodukte zu messen und wenigstens eine zugehörige Größe zu ermitteln, um wenigstens einen weiteren Bearbeitungsprozess vor und/oder hinter der Inspektionseinrichtung zu beeinflussen. Da erfindungsgemäß tolerierte Reste zurückbleiben, können diese gezielt zum Erfassen bzw. zur Messung vorgesehen werden, und die Inspektionseinrichtung, die als solche eine Mess- und Erfassungseinrichtung ist, wird gezielt auf die Textur des zu erfassenden Toleranzrestes eingestellt. Infolge dessen sind Toleranzreste ganz unterschiedlicher Natur und Erscheinungsform erfassbar, nämlich insbesondere solche, die Knochen, Knorpel, Blutgefäße, Fett, Haut, Membranen, Blasen, Sehnen, Gewebe, Parasiten, Einschlüsse, Struktur-Substanzabnormitäten und/oder Fremdkörper od. dgl. aufweisen. Zusätzlich zu der erfindungsgemäßen Einstellung und Nutzung zugelassener Toleranzreste kann eine besondere Ausgestaltung auch darin bestehen, dass eine oder mehrere zu inspizierende Texturen durch wenigstens einen Fleischbereich, wenigstens einen Schichtbereich, wenigstens einen Knochenbereich und/oder eine Mischstruktur solcher Bereiche bestimmt sind.

Um insbesondere die Abtrennung von tolerierten Resten an die Verhältnisse aufgrund einer Echtzeitmessung der Inspektionseinrichtung anzupassen, aber auch, um gegebenenfalls andere Bearbeitungsprozesse zu beeinflussen, hat es sich als besonders vorteilhaft erwiesen, Soll/Ist-Vergleiche durchzuführen, indem wenigstens eine Vergleichsgröße und/oder Relation von Vergleichsgrößen vorgegeben werden, um nach Maßgabe der Vorgabe(n) den jeweiligen gemessenen Bearbeitungsprozess zu beeinflussen. Zweckmäßig werden die Vorgabegrößen nach Maßgabe von Charakteristika des zu bearbeitenden Fleischproduktes bzw. Verzehrproduktes, Bearbeitungsparametem und/oder personellen Handhabungsparametern bestimmt.

Bei den Ist-Größen kann es sich um solche handeln, die mittels eines Rechners der Inspektionseinrichtung nach Maßgabe eines Programms und/oder Algorithmus berechnet werden, wobei zweckmäßig durchschnittliche Wertbildungen gemessener Werte einbezogen werden können. Die Durchschnittsbildung setzt die erfindungsgemäß zugelassenen Toleranzreste voraus.

Besonders vorteilhaft kann mit der Inspektionseinrichtung wenigstens eine den Grad der Verzehrproduktgewinnung (Ausbeute) repräsentierende Größe ermittelt und zum Optimieren der Gewinnung verwendet werden, indem die den Gewinnungsgrad repräsentierende Größe zum Steuern der Bearbeitung vor der Inspektionseinrichtung zum optimalen Verändern der genannten Größe verwendet wird. Im Beispiel einer Ausführungsform werden Fischprodukte enthäutet, wobei mittels der automatischen Inspektionseinrichtung die den Gewinnungsgrad nach dem Enthäuten repräsentierende Silber- oder Fettschicht gemessen und wenigstens eine entsprechende Größe ermittelt wird, die insbesondere nach Maßgabe vorgegebener Größe zum Einstellen des Enthäutens verwendet wird, um die Verzehrproduktgewinnung zu optimieren.

Zweckmäßig wird zum Minimieren der Toleranzreste der Verzehrprodukte wenigstens eine Texturinformations-Größe mittels eines Rechners der Inspektionseinrichtung ermittelt, der die Informations-Größen(n) aufgrund von Messungen berechnet. Als besonders vorteilhaft hat es sich erwiesen, dass mittels der Inspektionseinrichtung wenigstens zwei Texturinformations-Größen ermittelt werden, die zum Minimieren der Toleranzreste in Relation zueinander gesetzt werden, wobei zum Herbeiführen minimierter Toleranzreste wenigstens eine bestimmte Relation vorgegeben wird. Dieses Verfahren wird besonders vorteilhaft und bevorzugt dadurch ausgeführt, dass an mit minimiertem Toleranzrest behaftetem Verzehrprodukt eine den Toleranzrest repräsentierende Texturinformations-Größe und eine durch einen wenigstens teilweise von Toleranzresten freien Bereich bestimmte Texturinformations-Größe ermittelt werden. Dabei ist es zweckmäßig, dass in Bezug auf eine Produktart ein charakteristischer durchschnittlicher Produktanteil von Toleranzresten und ein durchschnittlicher restfreier Anteil in vorgegebener Verteilung für einen die Toleranzreste optimal minimierenden Trennvorgang in der Bearbeitungslinie vor der Inspektionseinrichtung bestimmt werden. Für eine solche Ausgestaltung ist die Inspektionseinrichtung mit einem Rechner eingerichtet, der Daten zur Verteilung von Toleranzresten nach Maßgabe der laufenden Messungen an den Verzehrprodukten berechnet. Zum Beispiel wird in der Inspektionseinrichtung berechnet, mit welchem durchschnittlichen Niveau in einem Geflügel-Verzehrprodukt Gabelbeinreste und mit welchen durchschnittlichen Niveau Fleisch zwischen Rippen (Rippenfleisch) auftritt. Es ist gefunden worden, dass bestimmte Niveauverhältnisse jeweils eine optimale Abtrennung von abzutrennenden Bestandteilen unter Belassen der Toleranzreste repräsentieren. Wenn zum Beispiel die Inspektionseinrichtung für eine Mehrzahl bearbeiteter Hühnerbrust-Filets durchschnittlich ein Niveau von 20 % an Gabelbeinresten und ein Niveau von 75 % an Rippenfleisch ermittelt hat, hat sich erwiesen, dass der Trennvorgang hinsichtlich höchster Ausbeute (Fleischgewinnung) optimal eingestellt worden ist. Im Rahmen der Erfindung wird eine solche optimale Einstellung als "Sweet Spot" bezeichnet. Eine der Erfindung zugrunde liegende Erkenntnis besteht also darin, dass eine optimale Ausbeute (Fleischgewinnung) beim Abtrennen vor der Inspektionseinrichtung erreicht wird, wenn man gezielt Knochenreste im Verhältnis zu dem gewonnenen Fleisch belässt bzw. toleriert. Da die Inspektionseinrichtung mit Mess-/Rechnermittel in der Lage ist, den tolerierten Rest sowie den Fleischanteil in höchstem Maße zuverlässig zu ermitteln, entstehen keine Nachteile, da die mit minimalen Resten behafteten Verzehrprodukte entsprechend sicher und zuverlässig erfasst und von den restfreien Verzehr-Filetprodukten separiert werden.

Der mit "Sweet Spot" bezeichnete erfindungsgemäße optimale Trennvorgang wird insbesondere in Abhängigkeit von der Produktgröße, von Bearbeitungsparametern, Handhabungspraktiken beim Beschicken und/oder von der Produktspezies (Schwarmspezies) oder dergleichen vorgegeben. Entsprechend ist der "Sweet Spot" von Fall zu Fall unterschiedlich, wobei er zum Gewinnen der Vorgabegröße empirisch ermittelt werden kann. Zum Beispiel wird das erwähnte 20 % zu 75 %-Verhältnis als Sollwert verwendet, mit dem der durchschnittliche Ist-Wert, gemessen und berechnet mittels der Inspektionseinrichtung, verglichen wird, um den Trennvorgang so zu beeinflussen und einzustellen, dass die höchste Effektivität ("Sweet Spot") erreicht wird.

Um den "Sweet Spot" gemäß besonders bevorzugter und zweckmäßiger erfindungsgemäßer Gestaltung selbsttätig herbeizuführen, wird das erfindungsgemäße Verfahren so ausgeführt, dass die Abtrennbestandteile der Fleischprodukte von diesen mittels einer automatischen Trenneinrichtung getrennt werden und dass die von der Inspektionseinrichtung gemessenen und ermittelten Größen in Rückkoppelungsschaltung verwendet werden, um den Trennvorgang zum Minimieren der Toleranzreste zu steuern.

Der "Sweet Spot" kann aber auch in Abhängigkeit von dem Personal bzw. einem Bearbeiter, die zum Beschicken der Filetier-Trenneinrichtung vor der Inspektionseinrichtung und/oder zur Trimmbearbeitung in der Produktlinie hinter der Inspektionseinrichtung eingesetzt werden, aufgrund einer unterschiedlichen Handhabung oder Handfertigkeit beeinflusst sein. Der "Sweet Spot" bzw. die ihm zugehörige optimal erreichbare Relation zwischen tolerierbarem Rest (zum Beispiel Gabelbeinreste) und gewonnenem Fleisch (zum Beispiel Rippenfleisch) wird also von Fall zu Fall ermittelt. Die Inspektionseinrichtung wird dann eingestellt, um den individuellen "Sweet Spot" - in Zuordnung zu Personal - einzustellen und beizubehalten. Zum Beispiel wird in einem Fall der tolerierbare Rest größer eingestellt als in einem anderen Fall, weil bei einer abschließenden manuellen Trimmbearbeitung im ersten Fall das Personal sorgfältiger arbeitet als im zweiten Fall. Bezogen auf die Produktausbeute über alle Bearbeitungsvorgänge ist dann die Ausbeute mit dem individuellen "Sweet Spot" für das Personal im zweiten Fall besser als bei einem Betrieb, bei dem die "Sweet Spot"-Einstellung wie im ersten Fall durchgeführt würde.

Das erfindungsgemäße Verfahren lässt sich besonders vorteilhaft gestalten und Ausführen, wenn von der Inspektionseinrichtung abgegebene restbehaftete und restfreie Verzehrprodukte automatisch voneinander separiert werden, indem die Inspektionseinrichtung eine Separatoreinrichtung steuert, die mit Toleranzresten behaftete Verzehrprodukte aus einem Strom von inspizierten Verzehrprodukten herausführt. Herausgeführte, mit Toleranzresten behaftete Verzehrprodukte können zum Abtrennen der Toleranzreste von Hand oder automatisch getrimmt und der Produktlinie, aus der sie herausgeführt worden sind, wieder zugeführt werden. Im Falle des automatischen Trimmens können die Messungen der Inspektionseinrichtung in Verbindung mit einer Rechner-Steuerung der Inspektionseinrichtung als Steuergrößen zum Trimmen verwendet werden.

Eine besondere Ausgestaltung der erfindungsgemäßen Verfahrens besteht auch darin, dass mittels der Inspektionseinrichtung im anatomischen Bereich eines Verzehrprodukts, z. B. eines Geflügelfilets das Vorhandensein von tolerierten Resten - z. B. von Gabelbein-Resten - und von Blut gemessen wird, um in diesem Bereich durch Schlachtung beeinflusste Eigenschaft des Produktes festzustellen und die Schlachtbearbeitung zu optimieren. Die Schlachtbearbeitung wird dann geändert bzw. dahingehend verbessert, dass das Auftreten von Hämatomen beseitigt und eine eventuelle Knochensplitterung vermieden werden.

Eine erfindungsgemäße Vorrichtung ist zweckmäßig mit einer Inspektionseinrichtung ausgestattet, die wenigstens ein Mess-/Steuermittel aufweist, das Toleranzreste oder andere Substanz-Texturstellen misst und entsprechende Anzeige- und/oder Steuergrößen ermittelt. Das genannte Mittel ist zweckmäßig mit einem elektronischen Rechner oder einer Prozessoreinheit ausgestattet, die nach Maßgabe eines Programms oder eines Algorithmus Steuergrößen berechnet. Parameter für solche Berechnungen oder Einstellungen dazu können insbesondere durch Art und/oder Eigenschaft der Reste, durch Verhältnisgrößen restbehafteter und restfreier Anteile, Bearbeitungsparameter und -informationen, zu denen auch Handhabungsmuster bzw. -fähigkeiten von Bedienpersonal gehören, bestimmt sein.

Ein Steuermittel bzw. ein Rechner dieses Steuermittels ist zweckmäßig auch so vorbereitet und geschaltet, dass wenigstens ein Bearbeitungsprozess durch einen Soll/IstVergleich gesteuert wird, wobei die Soll-Vorgabe insbesondere durch Charakteristika des zu bearbeitenden Fleischproduktes bzw. Verzehrproduktes und/oder Bearbeitungsparameter einschließlich Handhabungsbesonderheiten bestimmt ist.

Die Inspektionseinrichtung ist in bevorzugter Ausgestaltung mit einem Mess-/Steuermittel mit gegebenenfalls einem rechnergesteuerten Mittel derart eingerichtet, dass es Toleranzreste und damit in Kombination einen wenigstens teilweise von Toleranzresten freien Bereich der Verzehrprodukte zum Steuern der Trenneinrichtung vor der Inspektionseinrichtung hinsichtlich einer wahlweisen Minimierung zurückbleibender Toleranzreste feststellt. Vorzugsweise und zweckmäßig ist die Inspektionseinrichtung mit einem Mess-/Steuermittel ausgestattet, das Texturstellen in einer kombinierten Röntgen-/Optikmessung erfasst. Zum Beispiel werden mittels der Röntgenstrahlung nicht transparente Teile wie insbesondere nicht sichtbare verborgene Knochen und Knorpel erfasst, während ein optisches Messmittel transparente Texturen wie Fleisch, Bluteinschlüsse, Parasiten und dergleichen und gegebenenfalls sichtbare Knochen erfasst. Es ist gefunden worden, dass insbesondere mit einer Kombinationsmessung, die für unterschiedliche Texturstelle eingerichtete Messsensoren aufweist, eine im Wesentlichen 100 % ige Erkennung erreicht werden kann. Dies ist für das erfindungsgemäße Verfahren, das Verzehrprodukte/-stücke mit tolerierten Resten zulässt, zum Erfassen dieser Reste von besonderer Bedeutung.

In besonders vorteilhafter Ausgestaltung ist die Trenneinrichtung vor der Inspektionseinrichtung mit wenigstens einem durch die Inspektionseinrichtung steuerbaren Abtrennwerkzeug ausgestattet, wobei die Inspektionseinrichtung in Rückkoppelungsschaltung mit der Trenneinrichtung zum Minimieren der Toleranzreste nach Maßgabe eines Soll/Ist-Vergleichs verbunden ist. Zweckmäßig ist die Inspektionseinrichtung für den Soll/Ist-Vergleich so eingerichtet, dass die Steuerung nach Maßgabe einer vorgegebenen Relation zwischen restfreien und restbehafteten Teilen des Verzehrprodukts erfolgt.

Die Inspektionseinrichtung ist besonders vorteilhaft und zweckmäßig mit einer Separatoreinrichtung in einer Steuerschaltung verbunden, wobei die Separatoreinrichtung angeordnet und ausgebildet ist, um restbehaftete Verzehrprodukte aus inspiziertem Verzehrprodukt-Förderstrom herauszuführen. Die Separatoreinrichtung wird zweckmäßig so ausgestattet, dass sie zum Abtrennen von Toleranzresten in Verbindung mit wenigstens einer Trimmeinrichtung angeordnet und eingerichtet ist, wobei die Trimmeinrichtung von der Separatoreinrichtung separierte restbehaftete Verzehrprodukte empfängt und sie nach dem Trimmen zum Zurückführen in den Produktstrom der Vorrichtung bereitstellt. In Ausgestaltung kann die Trimmeinrichtung mit einem Mittel zum automatischen Abtrennen der Toleranzreste ausgestattet sein. Besonders vorteilhaft kann die automatische Trimmeinrichtung mittels Koppelungsschaltung mit der Inspektionseinrichtung derart verbunden sein, dass die Trimmabtrennung nach Maßgabe wenigstens einer von der Inspektionseinrichtung festgestellten Eigenschaft des Toleranzrestes, des Fleischproduktes und/oder des Verzehrproduktes erfolgt.

Unteransprüche sind auf die genannten und noch andere zweckmäßige und vorteilhafte Ausgestaltungen der Erfindung gerichtet. Besonders zweckmäßige und vorteilhafte Ausbildungsformen oder -möglichkeiten der Erfindung werden anhand der folgenden Beschreibung der in der schematischen Zeichnung dargestellten Ausführungsbeispiele näher beschrieben:
- Fig. 1 und 2: Diagramme zur Darstellung einer erfindungsgemäßen Vorrichtung und eines damit durchgeführten erfindungsgemäßen Verfahrens,
- Fig. 3: in axonometrischer Ansicht eine Bearbeitungslinie entsprechend der Diagrammdarstellung nach Fig. 1 und 2 und
- Fig. 4: ein Diagramm zur Darstellung von Bearbeitungsstationen in Verbindung mit einer automatischen Inspektionseinrichtung, die gemäß dem erfindungsgemäßen Verfahren betrieben und zur Steuerung von zusätzlichen Verfahrensabläufen im Zusammenhang mit erfindungsgemäßer "Sweet Spot"-Steuerung vorgesehen ist.

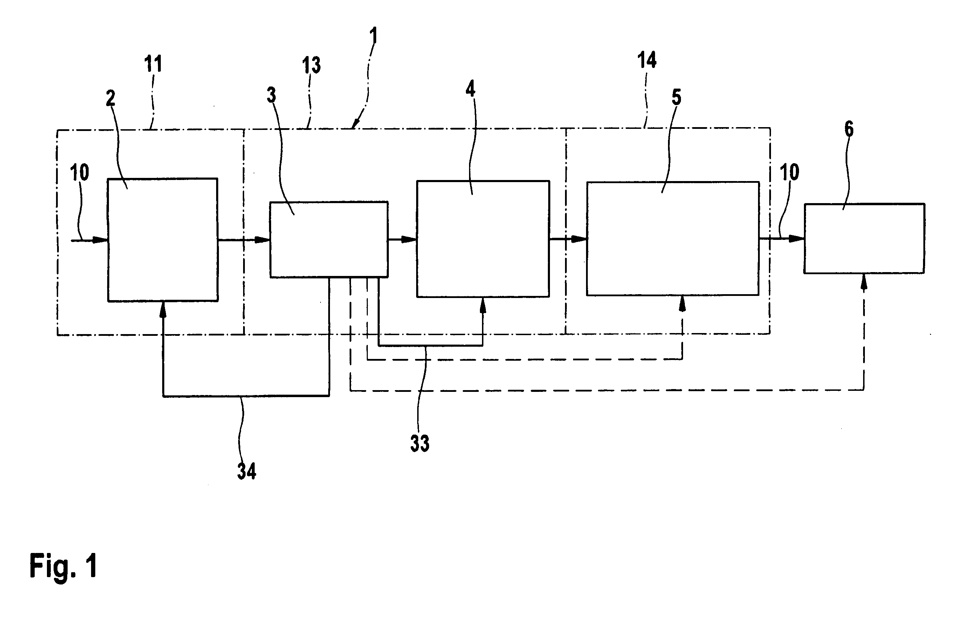

Wie aus Fig. 1, 2 und 3 ersichtlich, weist eine erfindungsgemäße Vorrichtung 1 entlang einer Bearbeitungslinie 10 Abschnitte 11, 12, 13 und 14 auf. Im Ausführungsbeispiel werden Geflügel-Vorderhälften filetiert. In einer an sich bekannten automatischen Trenneinrichtung 2 werden die Geflügel-Vorderhälften mit nicht dargestellten Halteeinrichtungen gehalten und transportiert. Vom Verzehr auszuschließende Bestandteile der Geflügel-Vorderhälfte werden dadurch abgetrennt, dass die zum Verzehr zu gewinnende Geflügelbrust unter Verwendung von nicht dargestellten computergesteuerten Schneidmessern und/oder Schabeklingen entfernt wird. Die gewonnenen Geflügelbrust-Verzehrprodukte 7 werden im Zuführabschnitt 12 auf einen Förderer 21 aufgegeben. Dort werden die Verzehrprodukte 7 (Filet-Geflügelbruststücke) auf horizontaler Bearbeitungsfläche derart platziert und ausgerichtet, dass sie nicht überlappen und die Schnittseiten nach oben gerichtet sind. Die Verzehrprodukte 7 werden so dann in dem Abschnitt 13 zunächst in eine Inspektionseinrichtung 3 längs der Linie 10 hineingefördert. Dort liegen die Verzehrprodukte 7 mit ihrer Schnittseite nach oben, so dass sie auf beim Trennen in der Trenneinrichtung 2 zurückgebliebene Reste von oben geprüft werden können.

In den Abschnitt 13 werden die inspizierten Verzehrprodukte 7 in zwei zur Bearbeitungs-/Transportlinie 10 parallelen, auf einem Förderer außen liegenden Reihen ausgegeben. Den beiden Transportreihen ist im Abschnitt 13 ein Separiermittel 4 zugeordnet, das durch eine Separatoreinrichtung 41 mit Auswurfarmen 410 gebildet ist. Das Separiermittel 4 ist über eine elektrische Koppelungsschaltung 33 mit der Inspektionseinrichtung 3 derart geschaltet und verbunden, dass Verzehrprodukte 72, die mit Defektresten behaftet sind, aus dem Strom von Verzehrprodukten 71, die die Inspektionseinrichtung 3 als restfrei erkannt hat, zu einer zusätzlichen Trimmbearbeitung, nämlich zum Abtrennen der Defektreste mittels einer Trimmeinrichtung 42 herausgeführt werden. Die derart getrimmten Verzehrprodukte 73 werden im Ausführungsbeispiel in eine Förderreihe zwischen den Reihen der Verzehrprodukte 71 wieder in die Bearbeitungslinie 10 eingeführt. Die anschließend in drei Reihen im Abschnitt 14 mit einem Förderer geförderten Verzehrprodukte 71 bzw. 73 werden einer Größensortier-Einrichtung 6 zugeführt, die die Verzehrprodukte 71, 73 automatisch nach ihrer Größe klassifiziert.

Erfindungsgemäß werden die Trenneinrichtung 2 und die dieser unmittelbar nachgeordnete Inspektionseinrichtung 3 derart betrieben, dass bei einer Anzahl der Verzehrprodukte 7 minimierte Reste zum weiteren Bearbeiten der Verzehrprodukte 71 zugelassen und herbeigeführt werden. Im Ausführungsbeispiel geht es darum, den Trennvorgang in der Trenneinrichtung 2 derart definiert nach Maßgabe der Inspektionsergebnisse der Trenneinrichtung 2 auszuführen, dass in einem definierten Maß Gabelbeinreste in den Verzehrprodukten (Geflügelbruststücken) 7 im Abschnitt 12 zugelassen werden, wobei die Inspektionseinrichtung zum Messen und Auswerten auf diese Reste eingerichtet und eingestellt wird.

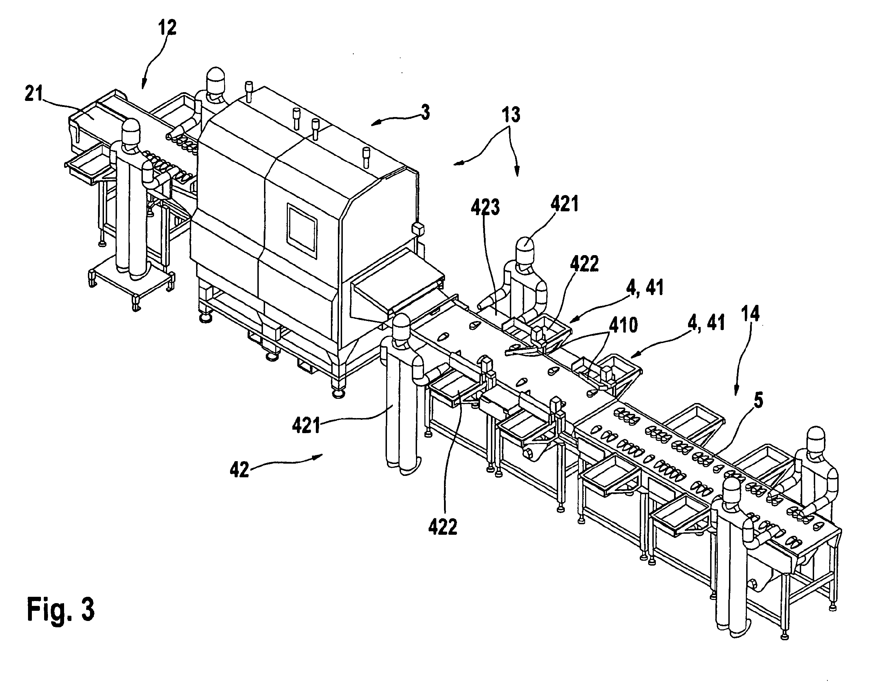

Im Abschnitt 14 kann eine weitere Kontrolle vorgesehen sein, indem dort eine manuelle Inspektion zum Feststellen von Verzehrprodukten mit Resten und ein manuelles Trimmen zum Abtrennen der Reste durchgeführt werden. Wie aus Fig. 3 ersichtlich, ist am Ende der Förderstrecke im Abschnitt 14 auch noch eine manuelle Endinspektion zum Feststellen von Verzehrprodukten mit Resten vorgesehen worden. Das Enderzeugnis wird dann zum anschließenden Abpacken zu der automatische Größensortiervorrichtung 6 gefördert.

Im Ausführungsbeispiel erreicht man erfindungsgemäß eine besondere Einstellung und Ausführung des Trennvorgangs in der Trenneinrichtung 2 dadurch, dass die Inspektionseinrichtung 3 aufgrund einer Kombinationsmessung Defektverteilungsdaten berechnet und diese Ergebnisse verwendet werden, um nach Maßgabe einer vorgegebenen Verteilung den Trennvorgang einzustellen, also eine Soll/Ist-Steuerung bzw. -Regelung vorzunehmen.

Die Inspektionseinrichtung 3 ist mit einem Messmittel 31 ausgestattet, das einerseits eine Sensoreinrichtung für verborgene, nicht sichtbare oder nicht durchscheinende Reste sowie andererseits eine Sensoreinrichtung für optisch erfassbare Reste aufweist. Im Ausführungsbeispiel ist das Messmittel 31 mit einer Röntgeneinrichtung ausgestattet, die nicht sichtbare, vollkommen verborgene Gabelbeinreste jedenfalls nahezu zu 100 % feststellt. Die optische Sensoreinrichtung erfasst Helligkeits- und/oder Farbunterschiede. Sie wird so ausgerichtet und eingestellt, dass sie den Anteil von knochenfreiem Fleisch, also dem Rippenfleisch in dem Geflügelbruststück ebenfalls wenigstens nahezu vollständig erfasst. Zweckmäßig wird die optische Messung zusätzlich eingestellt, um sichtbare oder durchscheinende Knochenreste zu messen. Erfindungsgemäß werden die Messungen an in Mehrzahl längs der Bearbeitungslinie 10 durch die Inspektionseinrichtung 3 transportierten Verzehrprodukten 7 derart durchgeführt, dass durch Auswertung und Verarbeitung der Messergebnisse in einem für sich bekannten programmgesteuerten Prozessrechner 32 Durchschnittswerte bzw. ein Durchschnittsverhältnis für die Anteile von Gabelbeinrest und Rippenfleisch erhalten wird.

Zum Beispiel ist gefunden worden, dass sich eine Hühnerbrust, die zum Filetieren von der Geflügel-Vorderhälfte abgetrennt wird, mit optimaler Qualität und Ausbeute filetieren lässt, wenn durchschnittlich ein Niveau von 20 % an Gabelbeinresten und ein Niveau von 75 % an Rippenfleisch gemessen wird. Der Trennvorgang wird dann erfindungsgemäß so eingestellt, dass diese durchschnittlichen Niveauverhältnisse auftreten bzw. beibehalten werden. Im Rahmen der Erfindung wird diese optimale Einstellung mit "Sweet Spot" bezeichnet. Die "Sweet Spot"-Einstellung kann empirisch ermittelt werden, und sie ist von einer Reihe von Faktoren abhängig, die durch das Fleischprodukt und Bearbeitungs- und/oder Handhabungsparameter oder -charakteristika bestimmt werden. Insbesondere kann die "Sweet Spot"-Einstellung von Verarbeitungsanlage zu Verarbeitungsanlage, also von Verwender zu Verwender unterschiedlich sein bzw. genau auf den Verwender und die Anlage abgestimmt und eingestellt werden. Die Einstellungen des Prozessrechners 32 der Trenneinrichtung 2 müssen zum Beispiel in Abhängigkeit von Geflügelgröße, Schwarmspezies und Handhabungspraktiken eingestellt werden. Zum Beispiel werden auch eine individuelle manuelle Bearbeitung oder Handhabung berücksichtigt. Insbesondere wird eine Arbeitsauslastung einbezogen, die in Abhängigkeit von Anzahl und/oder Qualifikation der bearbeitenden Personen längs der Bearbeitungslinie ermittelt wird.

Insbesondere ist gefunden worden, dass unter Verwendung eines bestimmten Verhältnisses aus Defektrest und defektfreiem Anteil eine Optimierung auch hinsichtlich von Resten erfolgen kann, die nicht zur Bestimmung des Restverhältnisses für den "Sweet Spot" herangezogen werden. Die erfindungsgemäß verwendete Inspektionseinrichtung 3 misst und ermittelt jede Art von Resten, die von dem Verzehrprodukt, im Ausführungsbeispiel also von dem Geflügelprodukt-Filet auszuschließen sind, also insbesondere auch Reste, die nicht zur minimalen tolerierbaren Resteinstellung verwendet werden. Dabei kommt es in jedem Fall wesentlich auf die Eignung der Inspektionseinrichtung 3 an, Reste bzw. diese bestimmende Texturstellen vollständig und jedenfalls mit nahezu 100 % -iger Sicherheit zu erfassen. Zu diesem Zweck wird das erwähnte Messsystem 3 eingesetzt, das Hybridmessungen ausführt, um ganz unterschiedliche Texturen gleichermaßen zuverlässig in einem gewünschten Zeitfenster zu erfassen.

Das erfindungsgemäße Verfahren ist nicht auf die Verwendung einer Inspektions-/-Messeinrichtung mit Hybrid-Messung eingeschränkt. Zum Beispiel kann es schon ausreichen, den Grad oder das Niveau eines tolerierten Restes mit einer Messart, z. B. mit einer Röntgenmessung zu erfassen. Soweit das Messsystem in der Lage ist, einen Rest ausreichend aufgrund des bloßen Auftretens ohne visuelle Erfassung zu ermitteln, müssen die zu prüfenden Verzehrprodukte zum Beschicken der Inspektionseinrichtung 3 nicht besonders ausgerichtet werden.

Mit Ermittlung der Festkörper-(Knochen-) oder anderer Rest-Anteile bzw. restfreier Anteile erhält man einen Maßstab für ein Knochenrisiko, das durch den Prozessrechner 32 der Inspektionseinrichtung 3 ermittelt wird. Selbstverständlich ist es Ziel, das Knochenrisiko zu minimieren. Erfindungsgemäß wird aber gezielt ein Knochenrisiko zugelassen, um damit Basis und Maßstab für die "Sweet Spot"-Optimierung zu erhalten.

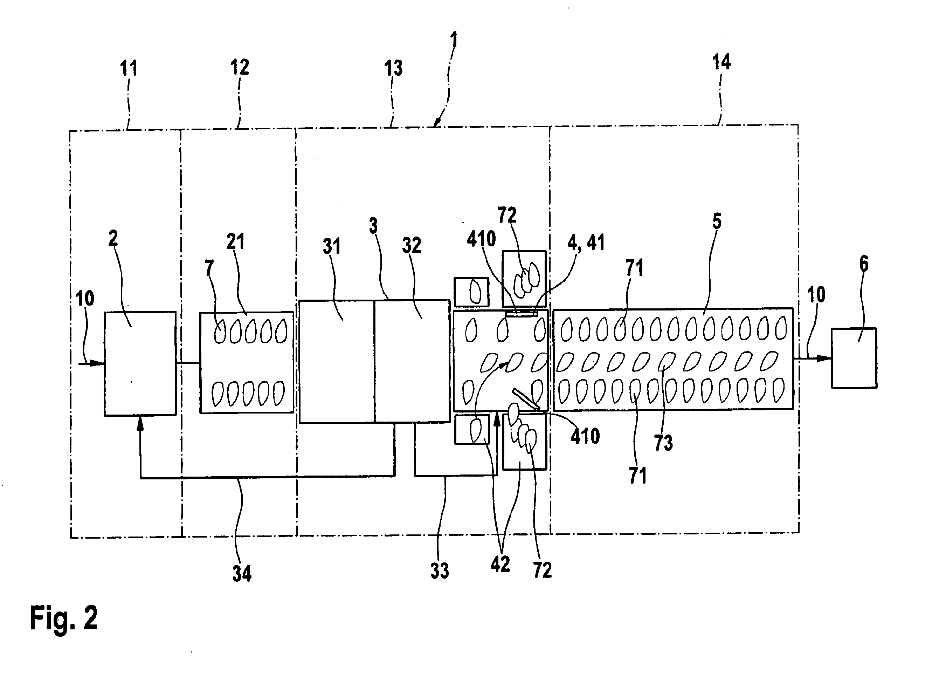

Der Prozessrechner 32 der Inspektionseinrichtung 3 wird zu dem vorstehend beschriebenen Soll/Ist-Vergleich mit den gewünschten charakteristischen bzw. anwendungsbezogenen Solldaten gespeist. Diese werden in den Rechner eingegeben. Im Ausführungsbeispiel der Fig. 1 und 2 ist die Trenneinrichtung 2 automatisiert und mittels der Inspektionseinrichtung 3 steuerbar. Zu diesem Zweck ist der Rechner 32 der Inspektionseinrichtung 3 in Rückkoppelungsschaltung 34 mit der Trenneinrichtung 2 verbunden. Auf diese Weise kann der Schnittvorgang oder jede andere zur Abtrennung geeignete, auch schnittfreie Trennung in der Trenneinrichtung 2 optimal an die tatsächlichen Gegebenheiten angepasst werden, um den "Sweet Spot" beizubehalten oder auch, um ihn mit geänderter Vorgabe neu einzustellen.

Aus der perspektivischen Ansicht der Fig. 3 in Verbindung mit der schematischen Draufsicht in Fig. 2 wird die im Ausführungsbeispiel dargestellte Bearbeitungslinie 10 mit den Abschnitten 11 bis 14 besonders deutlich. In Fig. 3 ist die Trenneinrichtung 2 nicht dargestellt. An dem Förderer 21 sieht man eine erste mit Personal besetzte Station, in der die zu bearbeitenden Verzehrprodukte 7, das heißt die Geflügelbruststücke zum Beschicken der Inspektions-/Auswertungseinrichtung 3 vorbereitet werden. Die Trimmeinrichtung 42 hinter der Inspektionseinrichtung 3 wird durch zwei Bedienungspersonen (Trimmer) 421 betrieben. Die Trimmstation weist vier Arbeitsplätze auf, von denen zwei besetzt sind. Die Separatoreinrichtung 41 ist mit Auswurfarmen 410 in Betrieb, die der besetzten Trimmstation zugeordnet sind. Mittels Steuerung über die Koppelungsschaltung 33 werden die defektbehafteten Verzehrprodukte 72 durch zeit- und ortgerechte Steuerung mittels der Arme 410, die in den Produktstrom quer eingreifen, in Behälter 422 geführt. Auf Trimmtischen 423 trennen die Trimmer 421 die Defektreste ab und führen die so gewonnenen Produkte zum Transport in die Mittelreihe des Förderers des Abschnitts 14. Eine solche Einreihung ist nur beispielhaft; die getrimmten Produkte können auch wieder in eine Reihe eingereiht werden, der sie entnommen worden sind. In Fig. 3 ist eine sich an die Trimmeinrichtung 42 anschließende Zwischen-Kontrollstation nicht mit Personal besetzt, während eine Endstation zur Endkontrolle mit zwei Personen besetzt ist. Von dieser Endstation werden die Verzehrprodukte, d. h. die filetierten Geflügelbruststücke der in Fig. 3 nicht dargestellten Größensortiereinrichtung 6 zugeführt.

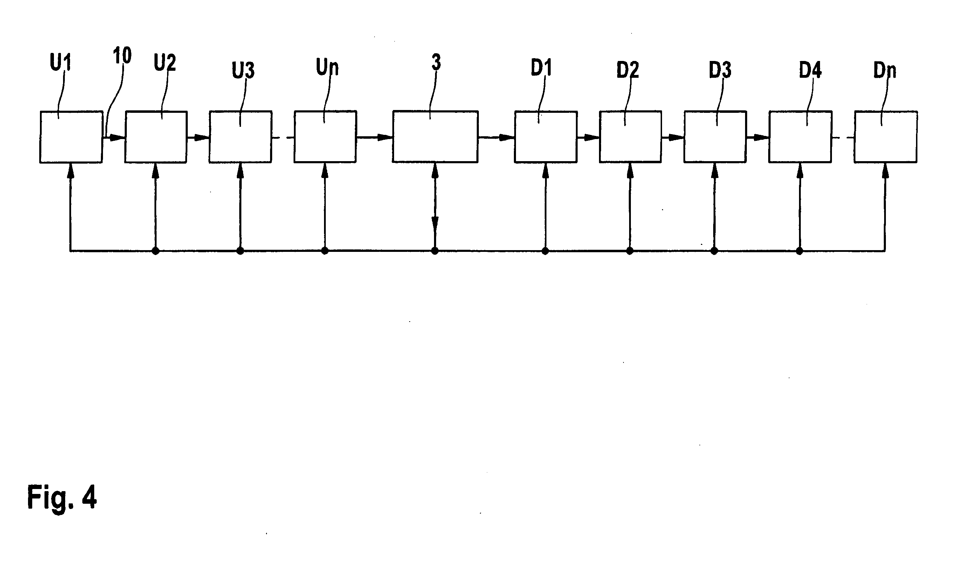

Erfindungsgemäß können auch Stationen, die wie die Trimmstationen in Fig. 3 durch Personal betrieben werden, aber auch andere Bearbeitungen vor und nach der Inspektionseinrichtung 3, also stromaufwärts bzw. stromabwärts automatisiert und nach Maßgabe von Mess- und Auswertungsergebnissen der Inspektionseinrichtung 3 von dieser gesteuert werden. Dies wird anhand der Fig. 4 erläutert.

In Fig. 4 sind längs einer Bearbeitungslinie 10 Bearbeitungsstationen U1, U2, U3, ... Un oberhalb der Inspektions-/Auswertungseinrichtung 3 sowie Bearbeitungsstationen D1, D2, D3, D4, ... Dn stromabwärts dargestellt. Dabei stehen Un und Dn jeweils für eine freie Anzahl.

Im Ausführungsbeispiel soll die Station U1 eine Schlachtstation für Geflügel darstellen, die Station U2 repräsentiert die Trenneinrichtung 2 zum Entbeinen gemäß dem vorstehend beschriebenen Ausführungsbeispiel und die Station U3 kann eine Station zum Entfernen einer Außenschicht von dem zu bearbeitenden Verzehrprodukt sein.

Die Stationen U1, U2, U3, ... Un sind jeweils in Rückkoppelungsschaltung mit der Inspektions-/Auswertungseinrichtung 3 verbunden, während die Stationen D1, D2, D3, D4, ... Dn jeweils in einer Vorwärtsschaltung mit der Inspektions-/Auswertungsschaltung 3 verbunden sind.

In Verbindung mit der erfindungsgemäßen "Sweet Spot"-Steuerung mittels der Station U2, wie anhand der Fig. 1 bis 3 beschrieben, kann auch der Schlachtvorgang optimiert werden. So ermittelt die Inspektions-/Auswertungseinrichtung 3 nicht nur den "Sweet Spot", sondern es wird mittels der Einrichtung 3 auch festgestellt, ob im anatomischen Bereich der Gabelbeinreste in dem Geflügelbruststück Blutreste auftreten, die beim Schlachten durch zerstörte Blutgefäße entstehen können. Zudem wird mit der Einrichtung 3 die Beschaffenheit der Gabelbeinreste ermittelt. Das Auftreten von Blutresten und/oder zertrümmerten Gabelbeinresten ist darauf zurückzuführen, dass der Schlachtvorgang zum Beispiel durch mangelnde Betäubung unbefriedigend ist. Die mittels der Inspektionseinrichtung 3 festgestellten Ergebnisse werden nun genutzt, um den Schlachtvorgang der Station U 1 zu optimieren.

Die Station U3 steht stellvertretend für einen weiteren Abtrenn-Bearbeitungsprozess zum Optimieren der Produktgewinnung und/oder der Qualitätsverbesserung. Das Abtrennen einer Schicht zur Filetgewinnung kann mit Bezug auf den nach der Schichtabtrennung verbleibenden, zum Vorschein kommenden Bereich genutzt werden. Wenn nämlich die dann zum Vorschein kommende Schicht den Gewinnungsgrad repräsentiert, so wird zweckmäßig mittels der Inspektions-/Auswertungseinrichtung 3 die verbleibende Schicht gemessen und dazu ein Wert berechnet, der zum Optimieren des Gewinnungsgrads zur Steuerung der Schichtabtrennung genutzt wird. Wenngleich ein solcher Prozess hier im Zusammenhang mit der Linie für ein Geflügelprodukt angesprochen wird, wird die Bedeutung einer solchen Bearbeitung besonders deutlich, wenn die zu bearbeitenden Produkte Fischfilets oder andere Fischprodukte sind, die enthäutet werden. Dann kann nämlich mittels der automatischen Inspektionseinrichtung die den Gewinnungsgrad nach dem Enthäuten repräsentierende Silber- oder Fettschicht gemessen und wenigstens eine entsprechende Größe ermittelt werden, die zweckmäßig nach Maßgabe vorgegebener Größe zum Einstellen des Enthäutens verwendet wird.

Die Station D1 ist bereits anhand der Fig. 1 bis 3 beschrieben worden. Diese Station ist in Kombination mit der beschriebenen "Sweet Spot"-Steuerung eine wesentliche Maßnahme, um Produkte, die die festgestellten Reste aufweisen, individuell zu bearbeiten und zu diesem Zweck automatisch aus dem Fluss der anderen Produkte herauszuführen.

Die individuelle Bearbeitung besteht insbesondere im Trimmen der ermittelten Verzehrprodukte und zweckmäßig auch im Zurückführen der getrimmten Verzehrprodukte in die Haupt-Bearbeitungslinie 10. Auch insoweit ist es vorteilhaft, dass auf Basis der erfindungsgemäßen "Sweet-Spot"-Steuerung und im Zusammenhang damit das Nachtrimmen und gegebenenfalls das Zurückführen der getrimmten Verzehrprodukte nach Maßgabe der mit der Inspektions-/Auswertungseinrichtung 3 festgestellten Verzehrprodukte bzw. der Inspektionsergebnisse durch die dargestellte Koppelungsschaltung automatisiert und gesteuert werden.

In Fig. 4 soll die Station D3 eine automatische Bearbeitung zur Endinspektion darstellen. So können Inspektionen im Abschnitt 14 des Ausführungsbeispiels gemäß Fig. 1 bis 3 automatisiert werden. Denkbar ist auch, dass von einer Inspektionseinrichtung der Station D4 gemessene und berechnete Daten bzw. Werte, die schließlich noch übrig gebliebene Defektreste erfassen, zu der Inspektions-/Auswertungseinrichtung 3 zurückgefiihrt und im Prozessrechner dieser Einrichtung 3 verarbeitet werden, um in der Station U2 den Trennvorgang weiter zu optimieren. Bei einer geeigneten Führung und Weiterleitung automatisch nachgetrimmter und wieder zurückgeführter Verzehrprodukte können die Auswertungsergebnisse der Einrichtung 3 auch dazu genutzt werden, gezielt in Bezug auf nachgetrimmte Verzehrstücke oder eine definierte Gruppe bzw. Charge von Verzehrstücken und/oder bei Feststellung erhöhter Häufigkeit mittels der Einrichtung 3 wenigstens eine weitere automatische Inspektion durchzuführen.

In Fig. 4 soll die Station D4 eine Einrichtung sein, die die Verzehrstücke automatisch zum Beispiel nach ihrem Gewicht portioniert und sortiert. Auch dazu können die mit der Inspektions-/Auswertungseinrichtung 3 ermittelten Daten herangezogen werden, indem mit der Einrichtung 3 die Produktgröße und/oder das Produktgewicht betreffende Werte der Verzehrprodukte ermittelt und zur automatischen Steuerung der Sortiereinrichtung, zum Beispiel der Sortiereinrichtung 6 verarbeitet und genutzt werden. Die Verbindungen und Kopplungen der Inspektions-/Auswertungseinrichtung 3 mit den automatisierten Station U1, U2, ... Un bzw. D1, D2, ... Dn können wie im Beispiel der Station D3 auch derart sein, dass von den Stationen Daten an die Einrichtung 3 gegeben werden, um Steuerungsvorgänge mit Rückmeldungen gesteuerter/steuernder Stationen durchzuführen.

Man erkennt, dass die Inspektions-/Auswertungseinrichtung 3 in Verbindung mit der Durchführung der Messung und Auswertung zu dem "Sweet Spot" zur Steuerung einer Reihe von Bearbeitungsstationen einsetzbar ist. Eine wesentliche Grundlage dafür ist, dass die Inspektions-/Auswertungseinrichtung erfindungsgemäß vorgesehen wird, um beim Abtrennen zurückbleibende Reste zur Messung und Auswertung besonders zu definieren und vorzubereiten, indem sie in definiertem Maß zugelassen und herbeigeführt werden.

## Patentansprüche

1. Verfahren zum Bearbeiten von in Mehrzahl entlang einer Bearbeitungslinie (10) geförderten Fisch-, Geflügel- oder anderen Fleischprodukten jeder Art und Form, die nicht verzehrbare oder vom Verzehr auszuschließende abzutrennende Bestandteile, sogenannte Abtrennbestandteile, wenigstens einer Art enthalten, wobei die Abtrennbestandteile von den Fleischprodukten getrennt werden und **dadurch** zum Verzehr gewonnene Verzehrprodukte (7) mittels einer automatischen Inspektionseinrichtung (3) auf beim Trennen zurückgebliebene Reste geprüft werden, umfassend folgende Schritte:
i) Die Abtrennbestandteile werden von den Fleischprodukten derart getrennt, dass bei einer Anzahl der Verzehrprodukte (7) minimierte Reste als Toleranzreste wenigstens einer Art zum weiteren Bearbeiten der Verzehrprodukte (7), insbesondere zum Optimieren der Verzehrproduktgewinnung, zugelassen und herbeigeführt werden,
ii) die Toleranzreste werden in einem Maß eingestellt, durch das das Auftreten von die Toleranzreste aufweisenden Verzehrprodukten (7) hinsichtlich gewünschter Bearbeitung mit einer Häufigkeit eingestellt und belassen wird, durch die der Trennvorgang insbesondere in Abhängigkeit von der Produktgröße, von Bearbeitungsparametern, Handhabungspraktiken beim Beschicken und/oder von der Produktspezies vorgegeben wird,
iii) die automatische Inspektionseinrichtung (3) wird zum Feststellen der nach Schritt ii) eingestellten, zurückbleibenden Toleranzreste eingestellt und
iv) von der Inspektionseinrichtung (3) abgegebene und mit den Toleranzresten behaftete und restfreie Verzehrprodukte (71, 72) werden separiert.

2. Verfahren nach Anspruch 1, **dadurch gekennzeichnet, dass**die von der automatischen Inspektionseinrichtung (3) festgestellten und von ihr abgegebenen, Toleranzreste aufweisenden Verzehrprodukte (71) getrimmt werden, indem Toleranzreste vorzugsweise manuell abgetrennt werden.

3. VerfahrennachAnspruch2, **dadurch gekennzeichnet, dass** die getrimmten Verzehrprodukte (73) sowie von der Inspektionseinrichtung (3) als restfrei festgestellte und abgegebene Verzehrprodukte (71) einer gemeinsamen Bearbeitungslinie (10) zugeführt werden.

4. Verfahren nach Anspruch 3, **dadurch gekennzeichnet, dass** im Bereich der gemeinsamen Bearbeitungslinie (10) zusätzlich eine manuelle Inspektion zum Feststellen von Toleranzresten und ein manuelles Trimmen zum Abtrennen von Toleranzresten durchgeführt werden.

5. Verfahren nach Anspruch 3 oder 4, **dadurch gekennzeichnet, dass** am Ende der gemeinsamen Bearbeitungslinie eine manuelle oder automatische Endinspektion zum Feststellen von Toleranzresten durchgeführt wird.

6. Verfahren nach einem der Ansprüche 1 bis 5, **dadurch gekennzeich**n e t, dass mittels der Inspektionseinrichtung (3) wenigstens eine Toleranzreste repräsentierende Textur und gegebenenfalls wenigstens eine weitere Substanz-Textur der Verzehrprodukte gemessen werden und wenigstens eine Größe ermittelt wird, die eine qualitative, quantitative und/oder örtliche Textur-Substanzinformation der Verzehrprodukte (7) repräsentiert und zum Beeinflussen wenigstens eines Abtrennprozesses vor und gegebenenfalls wenigstens eines weiteren Bearbeitungsprozesses (U, D) vor und/oder hinter der Inspektionseinrichtung (3) verwendet wird.

7. Verfahren nach Anspruch 6, **dadurch gekennzeichnet, dass** eine oder mehrere zu inspizierende Texturen bzw. abzutrennende Texturstellen durch Toleranzreste bestimmt sind, die Knochen, Knorpel, Blutgefäße, Fett, Haut, Membranen, Blasen, Sehnen, Gewebe, Parasiten, Einschlüsse, Struktur-/Substanzabnormitäten, Fremdkörper od. dgl. enthalten.

8. Verfahren nach Anspruch 6 oder 7, **dadurch gekennzeichnet, dass** eine oder mehrere zu inspizierende Texturen durch wenigstens einen Fleischbereich, wenigstens einen Schichtbereich, wenigstens einen Knochenbereich und/oder eine Mischstruktur solcher Bereiche bestimmt sind.

9. Verfahren nach einem der Ansprüche 6 bis 8, **dadurch gekennzeichnet, dass** wenigstens eine Vergleichsgröße und/oder Relation von Vergleichsgrößen vorgegeben werden, um nach Maßgabe der Vorgabe(n) wenigstens einen Bearbeitungsprozess (U, D) zu beeinflussen.

10. VerfahrennachAnspruch9, **dadurch gekennzeichnet, dass** die Vorgabegröße(n) nach Maßgabe von Charakteristika des zu bearbeitenden Fleischproduktes, Verzehrproduktes (7) und/oder nach Bearbeitungsparametern bestimmt werden.

11. Verfahren nach einem der Ansprüche 6 bis 10, **dadurch gekennzeichne**t, dass mit der Inspektionseinrichtung (3) wenigstens eine den Grad der Verzehrproduktgewinnung, Ausbeute, repräsentierende Größe ermittelt und zum Optimieren der Gewinnung verwendet wird, indem die den Gewinnungsgrad repräsentierende Größe zum Steuern der Bearbeitung vor der Inspektionseinrichtung zum optimalen Verändern der genannten Größe verwendet wird.

12. Verfahren nach Anspruch 11, **dadurch gekennzeichnet, dass** die zu bearbeitenden Produkte Fischprodukte sind, die enthäutet werden, und dass mittels der automatischen Inspektionseinrichtung (3) die den Gewinnungsgrad nach dem Enthäuten repräsentierende Silber- oder Fettschicht gemessen und wenigstens eine entsprechende Größe ermittelt wird, die insbesondere nach Maßgabe vorgegebener Größe zum Einstellen des Enthäutens verwendet wird, um die Verzehrproduktgewinnung zu optimieren.

13. Verfahren nach einem der Ansprüche 6 bis 12, **dadurch gekennzeichnet, dass** wenigstens eine Texturinformations-Größe ermittelt wird, um beim Abtrennen der Abtrennbestandteile von den Fleischprodukten die Toleranzreste zu minimieren.

14. Verfahren nach Anspruch 13, **dadurch gekennzeichnet, dass** wenigstens zwei Texturinformations-Größen ermittelt werden, die zum Minimieren der Toleranzreste in Relation zueinander gesetzt werden, wobei zum Herbeiführen minimierter Toleranzreste wenigstens eine bestimmte Relation vorgegeben wird.

15. Verfahren nach Anspruch 14, **dadurch gekennzeichnet, dass** an mit minimiertem Toleranzrest behaftetem Verzehrprodukt (7, 71) eine den Toleranzrest repräsentierende Texturinformations-Größe und eine durch einen wenigstens teilweise von Toleranzresten freien Bereich bestimmte Texturinformations-Größe ermittelt werden.

16. Verfahren nach Anspruch 15, **dadurch gekennzeichnet, dass** in Bezug auf eine Produktart ein charakteristischer durchschnittlicher Produktanteil von Toleranzresten und ein durchschnittlicher restfreier Anteil in vorgegebener Verteilung für einen die Toleranzreste optimal minimierenden Trennvorgang bestimmt werden.

17. Verfahren nach einem der Ansprüche 1 bis 16, **dadurch gekennzeichnet, dass** die Abtrennbestandteile der Fleischprodukte von diesen mittels einer automatischen Trenneinrichtung (2) getrennt werden und dass die von der Inspektionseinrichtung (3) gemessenen bzw. ermittelten Größen in Rückkoppelungsschaltung (32) verwendet werden, um den Trennvorgang zum Minimieren der Toleranzreste zu steuern.

18. Verfahren nach einem der Ansprüche 6 bis 17, **dadurch gekennzeichnet, dass** von der Inspektionseinrichtung (3) abgegebene restbehaftete und restfreie Verzehrprodukte (71, 72) automatisch voneinander separiert werden, indem die Inspektionseinrichtung (3) eine Separator-Einrichtung (41) steuert, die mit Toleranzresten behaftete Verzehrprodukte (72) aus einem Strom von inspizierten Verzehrprodukten herausführt.

19. Verfahren nach Anspruch 18, **dadurch gekennzeichnet, daß** die inspizierten Verzehrprodukte (7) von der Inspektionseinrichtung (3) in wenigstens einer Reihe und vorzugsweise in zwei parallelen Reihen abgegeben werden.

20. Verfahren nach Anspruch 19, **dadurch gekennzeichnet, dass** herausgeführte, mit Toleranzresten behaftete Verzehrprodukte (72) zum Abtrennen der Toleranzreste getrimmt werden und die getrimmten Verzehrprodukte (73) in wenigstens einer Reihe der Produktlinie (10) wieder zugeführt werden.

21. Verfahren nach einem der Ansprüche 6 bis 20, **dadurch gekennzeichnet, dass** mit Toleranzresten behaftete Verzehrprodukte (71) zum Abtrennen der Toleranzreste automatisch getrimmt werden, indem die Inspektionseinrichtung (3) eine Trimmeinrichtung (42) steuert.

22. Verfahren nach einem der Ansprüche 1 bis 21, **dadurch gekennzeichnet, dass** Geflügel-Vorderhälften oder -Brustkappen bearbeitet und abgetrennte Filets mittels der automatischen Inspektionseinrichtung (3) inspiziert werden, wobei zum Optimieren des Trennvorgangs der als Toleranzrest verbleibende Anteil von Gabelbeinen und der als Toleranzrest verbleibende Anteil von Rippenfleisch gemessen und die ermittelten Größen in vorgegebene Relation gesetzt werden.

23. Verfahren nach einem der Ansprüche 1 bis 22, **dadurch gekennzeichnet, dass** mittels der Inspektionseinrichtung (3) im anatomischen Bereich eines Verzehrprodukts das Vorhandensein von tolerierten Resten und von Blut gemessen wird, um in diesem Bereich durch Schlachtung beeinflusste Eigenschaft des Produktes festzustellen und die Schlachtbearbeitung zu optimieren.

24. Vorrichtung (1) zum Bearbeiten von in Mehrzahl entlang einer Bearbeitungslinie (10) geförderten Fisch-, Geflügel- oder anderen Fleischprodukten jeder Art und Form zum Durchführen des Verfahrens nach einem der Ansprüche 1 bis 23, umfassend eine Trenneinrichtung (2) zum Abtrennen von nicht verzehrbaren oder vom Verzehr auszuschließenden Bestandteilen, sogenannte Abtrennbestandteile, eine der Trenneinrichtung (2) nachgeordnete automatische Inspektionseinrichtung (3) zum Prüfen der durch die Abtrennung gewonnenen Verzehrprodukte (7) auf zurückgebliebene Reste und ein der Inspektionseinrichtung (3) nachgeordnetes, eine Seperatoreinrichtung (41) umfassendes Mittel (4) zum Separieren restbehafteter und restfreier Verzehrprodukte (71, 72), die nach Maßgabe der Inspektionsergebnisse der Inspektionseinrichtung (3) unterschieden und voneinander getrennt abgegeben werden, wobei die Trenneinrichtung (2) mit wenigstens einem Abtrennwerkzeug eingerichtet ist und die automatische Inspektionseinrichtung (3) zum Feststellen restbehafteter Verzehrprodukte (71, 72) eingerichtet ist, **dadurch gekennzeichnet, dass** die Inspektionseinrichtung (3) wenigstens ein Mess- und Steuermittel (31) aufweist, das nach Maßgabe von Qualität, Quantität und/oder der Stelle des Auftretens die mittels des Verfahrens nach einem der Ansprüche 1 bis 23 mit vorgegebener Häufigkeit eingestellten Toleranzreste wenigstens einer Art misst, die durch die Einrichtung des wenigstens einen Abtrennwerkzeuges bei einer Anzahl der Verzehrprodukte (7) zum weiteren Bearbeiten der Verzehrprodukte (7), insbesondere zum Optimieren der Verzehrproduktgewinnung, über den Arbeitsablauf mit der eingestellten Häufigkeit verteilt zurückbleiben, wobei die automatische Inspektionseinrichtung (3) zum Feststellen dieser Toleranzreste derart eingerichtet ist, dass das Mess- und Steuermittel (31) wenigstens eine das Messergebnis gemäß eingestellter Häufigkeit repräsentierende Größe zum Steuern wenigstens eines Abtrenn-Bearbeitungsprozesses vor der Inspektionseinrichtung (3) erzeugt.

25. Vorrichtung nach Anspruch 24, **dadurch gekennzeichnet, dass** das wenigstens eine Mess- und Steuermittel (31) wenigstens eine das Messergebnis repräsentierende Größe zum Steuern wenigstens eines weiteren Bearbeitungsprozesses vor und/oder hinter der Inspektionseinrichtung (3) erzeugt.

26. Vorrichtung nach Anspruch 24 oder 25, **dadurch gekennzeichnet, dass** das wenigstens eine Mess- und Steuermittel (31) der Inspektionseinrichtung (3) zum Messen wenigstens einer durch den Toleranzrest entstehenden Substanz-Texturstelle der Verzehrprodukte sowie zum Erzeugen einer entsprechenden Größe eingerichtet ist, wobei wenigstens eine Substanz-Texturstelle insbesondere durch Reste, die Knochen, Knorpel, Blutgefäße, Fett, Haut, Membranen, Blasen, Sehnen, Gewebe, Parasiten, Einschlüsse, Struktur-/Substanzabnormitäten, Fremdkörper od. dgl. aufweisen, kenntlich ist.

27. Vorrichtung nach einem der Ansprüche 24 bis 26, **dadurch gekennzeichnet, dass** das wenigstens eine Mess- und Steuermittel (31) der Inspektionseinrichtung (3) zum Messen wenigstens einer Textur der Verzehrprodukte sowie zum Erzeugen einer entsprechenden Größe eingerichtet ist, wobei die Textur durch wenigstens einen Fleischbereich, wenigstens einen Schichtbereich, wenigstens einen Knochenbereich und/oder eine Mischstruktur solcher Bereiche kenntlich ist.

28. Vorrichtung nach einem der Ansprüche 25 bis 27, **dadurch gekennzeichnet, dass** wenigstens ein Steuermittel (31, 32, 33, 34) der Inspektionseinrichtung (3) die Steuergröße und/oder Steuergrößen, die durch wenigstens eine Relation bestimmt sind, zum Steuern wenigstens eines Bearbeitungsprozesses nach wenigstens einer Vorgabe erzeugt, die durch Charakteristika des zu bearbeitenden Fleischprodukts, Verzehrprodukts (7) und/oder Bearbeitungsparameter bestimmt sind.

29. Vorrichtung nach einem der Ansprüche 25 bis 28, **dadurch gekennzeichnet, dass** das wenigstens eine Mess- und Steuermittel (31) der Inspektionseinrichtung (3) derart eingerichtet ist, dass es die Toleranzreste und damit in Kombination einen wenigstens teilweise von den Toleranzresten freien Bereich der Verzehrprodukte (7) zum Steuern der Trenneinrichtung (2) vor der Inspektionseinrichtung (3) hinsichtlich der Minimierung zurückbleibender Toleranzreste feststellt.

30. Vorrichtung nach Anspruch 29, **dadurch gekennzeichnet, dass** das Kombinations-Mess- und Steuermittel (31) ein Röntgen-Messmittel und ein optisches Messmittel umfasst.

31. Vorrichtung nach einem der Ansprüche 24 bis 30, **dadurch gekennzeichnet, dass** die Trenneinrichtung (2) vor der Inspektionseinrichtung (3) mit wenigstens einem durch die Inspektionseinrichtung (3) steuerbaren AbtrennWerkzeug ausgestattet ist und dass die Inspektionseinrichtung (3) in Rückkoppelungsschaltung (34) mit der Trenneinrichtung (2) zum Minimieren der Toleranzreste nach Maßgabe eines Soll/Ist-Vergleichs verbunden ist.

32. Vorrichtung nach einem der Ansprüche 24 bis 31, **dadurch gekennzeichnet, dass** das Separatormittel (4) hinter der Inspektionseinrichtung (3) zum Separieren von von den Toleranzresten freien und restbehafteten Verzehrprodukten (7) der Separatoreinrichtung (41) aufweist, die restbehaftete Verzehrprodukte (72) aus inspiziertem Verzehrprodukt-Förderstrom herausführt.

33. Vorrichtung nach Anspruch 32, **dadurch gekennzeichnet, dass** die Separatoreinrichtung (41) hinter der Inspektionseinrichtung (3) zum Abtrennen von den Toleranzresten in Verbindung mit wenigstens einer Trimmeinrichtung (42) angeordnet und eingerichtet ist, die von der Separatoreinrichtung (41) separierte restbehaftete Verzehrprodukte (72) empfängt und sie nach dem Trimmen zum Zurückführen in den Produktstrom der Vorrichtung (1) bereitstellt.

34. Vorrichtung nach Anspruch 33, **dadurch gekennzeichnet, dass** die Separator-Trimmeinrichtung (42) mit einem Mittel zum automatischen Abtrennen der Toleranzreste ausgestattet ist.

35. Vorrichtung nach Anspruch 34, **dadurch gekennzeichnet, dass** die automatische Trimmeinrichtung (42) mittels Koppelungsschaltung mit der Inspektionseinrichtung (3) derart verbunden ist, dass die Trimmabtrennung nach Maßgabe wenigstens einer von der Inspektionseinrichtung (3) festgestellten Eigenschaft des Toleranzrestes, des Fleischproduktes und/oder des Verzehrproduktes (7) erfolgt.

## Claims

1. Method for the processing of fish, poultry or other meat products of any kind and shape being conveyed in a plurality along a processing line (10), which contain inedible components or components which are to be excluded from consumption and separated, so-called components to be separated, of at least one kind, in which the components to be separated are separated from the meat products, and edible products (7) obtained as a result for consumption are checked with an automatic inspection device (3) for residues left behind during separation, comprising the following steps:
i) the components to be separated are separated from the meat products in such a way that, with a number of edible products (7), minimised residues of at least one kind are permitted and induced for further processing of the edible products (7), in particular to optimise the edible product recovery;
ii) the tolerance residues are adjusted to an extent, by means of which the appearance of edible products (7) having tolerance residues, with regard to desired processing, are adjusted and retained with a frequency, by means of which the separation process is determined in particular depending on the product size, process parameters, handling methods during loading and/or product species,
iii) the automatic inspection device (3) is adjusted to detect the remaining tolerance residues adjusted according to step ii) and
iv) edible products (71, 72) discharged by the inspection device (3) with adhering tolerance residues and residue-free are separated.

2. Method according to claim 1, **characterised in that** the edible products (71) having tolerance residues and detected by the automatic inspection device (3) and discharged by it are trimmed, by preferably manually separating tolerance residues.

3. Method according to claim 2, **characterised in that** the trimmed edible products (73) as well as edible products (71) detected as residue-free and discharged by the inspection device (3) are delivered to a common processing line (10).

4. Method according to claim 3, **characterised in that** in the region of the common processing line (10) in addition manual inspection for the detection of tolerance residues and manual trimming for the separation of tolerance residues are performed.

5. Method according to claim 3 or 4, **characterised in that** at the end of the common processing line a manual or automatic final inspection is performed for detecting tolerance residues.

6. Method according to any of claims 1 to 5, **characterised in that**, by means of the inspection device (3), at least one texture representing tolerance residues and if necessary at least one further substance texture of the edible products are measured, and at least one variable which represents qualitative, quantitative and/or local texture substance information of the edible products (7) and is used to influence at least one separating process in front and if necessary at least one further processing step (U, D) in front of and/or behind the inspection device (3), is determined.

7. Method according to claim 6, **characterised in that** one or more textures to be inspected or texture areas to be separated are determined by tolerance residues which contain bones, gristle, blood vessels, fat, skin, membranes, bubbles, sinews, tissues, parasites, inclusions, structure/substance abnormalities, foreign bodies or the like.

8. Method according to claim 6 or 7, **characterised in that** one or more textures to be inspected are determined by at least one meat area, at least one layer area, at least one bone area and/or a structure combining such areas.

9. Method according to any of claims 6 to 8, **characterised in that** at least one reference variable and/or ratio of reference variables are predetermined in order to influence at least one processing step (U, D) according to the standard(s).

10. Method according to claim 9, **characterised in that** the preset variables are determined according to characteristics of the meat product, edible product (7) to be processed and/or according to processing parameters.

11. Method according to any of claims 6 to 10, **characterised in that** with the inspection device (3) at least one variable representing the degree of edible product recovery, yield, is determined and used to optimise recovery, by using the variable which represents the degree of recovery to control processing before the inspection device for optimally varying the aforementioned variable.

12. Method according to claim 11, **characterised in that** the products to be processed are fish products which are skinned, and **in that** the silver or fat layer representing the degree of recovery after skinning is measured with the automatic inspection device (3), and at least one corresponding variable is determined, which is used particularly according to a preset variable for adjustment of skinning to optimise edible product recovery.

13. Method according to any of claims 6 to 12, **characterised in that** at least one texture information variable is determined to minimise the tolerance residues during separation of the components to be separated from the meat products.

14. Method according to claim 13, **characterised in that** at least two texture information variables are determined, which are put in relation to each other to minimise the tolerance residues, at least one given ratio being preset to induce minimised tolerance residues.

15. Method according to claim 14, **characterised in that** a texture information variable representing the tolerance residue and a texture information variable defined by an area which is at least partially free from tolerance residues, are determined on edible product (7, 71) with minimised tolerance residue.

16. Method according to claim 15, **characterised in that**, with respect to one kind of product, a characteristic average product fraction of tolerance residues and an average residue-free fraction are defined in a preset distribution for a separating process which minimises the tolerance residues optimally.

17. Method according to any of claims 1 to 16, **characterised in that** the components of the meat products to be separated are separated from the latter with an automatic separating device (2), and **in that** the variables measured or determined by the inspection device (3) are used in a feedback circuit (32) to control the separating process to minimise the tolerance residues.

18. Method according to any of claims 6 to 17, **characterised in that** edible products (71, 72) with and without residues discharged by the inspection device (3) are separated from each other automatically by the fact that the inspection device (3) controls a separator device (41) which extracts edible products (72) with tolerance residues from a stream of inspected edible products.

19. Method according to claim 18, **characterised in that** the inspected edible products (7) are discharged by the inspection device (3) in at least one row and preferably in two parallel rows.

20. Method according to claim 19, **characterised in that** extracted edible products (72) with tolerance residues are trimmed to separate the tolerance residues and the trimmed edible products (73) are returned to the product line (10) in at least one row.

21. Method according to any of claims 6 to 20, **characterised in that** edible products (71) with tolerance residues are trimmed automatically to separate the tolerance residues, by the fact that the inspection device (3) controls a trimming device (42).

22. Method according to any of claims 1 to 21, **characterised in that** front halves of poultry or breast caps are processed, and separated fillets are inspected with the automatic inspection device (3), the fraction of wishbones remaining as the tolerance residue and the fraction of rib meat remaining as the tolerance residue being measured, and the variables determined being put in a preset ratio, to optimise the separating process.

23. Method according to any of claims 1 to 22, **characterised in that** the presence of tolerated residues and blood is measured with the inspection device (3) in the anatomical region of an edible product, in order to detect properties of the product which are influenced in this region by slaughter and optimise slaughter processing.

24. Apparatus (1) for processing fish, poultry or other meat products of any kind and shape conveyed in a plurality along a processing line (10), for carrying out the method according to any of claims 1 to 23, comprising a separating device (2) for separating inedible components or components which are to be excluded from consumption, so-called components to be separated, an automatic inspection device (3) mounted downstream the separating device (2) for checking the edible products (7) obtained by separation for residues left behind, and a means (4) including a separator device (41) mounted downstream the inspection device (3) for separating edible products (71, 72) with and without residues, which are distinguished according to the inspection results of the inspection device (3) and discharged separately from each other, wherein the separating device (2) is designed with at least one separating tool and the automatic inspection device (3) is designed to detect edible products (71, 72) with residues, **characterised in that** the inspection device (3) has at least one measuring and control means (31) which, according to quality, quantity and/or location of occurrence, measures the tolerance residues of at least one kind adjusted with predetermined frequency by means of the method according to any one of claims 1 to 23, which tolerance residues, by means of the arrangement of the at least one separating tool, are left behind with a number of edible products (7) for further processing of these edible products (7), in particular to optimise the edible product recovery, over the entire work cycle with the adjusted frequency, wherein the automatic inspection device (3) is designed to detect the tolerance residues in such a way that the measuring and control means (31) generates at least one variable representing the measurement result according to adjusted frequency for the control of at least one separating-processing step upstream of the inspection device (3).

25. Apparatus according to claim 24, **characterised in that** the at least one measuring and control means (31) generates at least one variable representing the measurement result for the control of at least one further processing step upstream and/or downstream of the inspection device (3).

26. Apparatus according to claim 24 or 25, **characterised in that** the at least one measuring and control means (31) of the inspection device (3) is designed for measuring at least one substance texture area of the edible products arising due to the tolerance residue and for generating a corresponding variable, at least one substance texture area being recognisable in particular by residues which comprise bones, gristle, blood vessels, fat, skin, membranes, bubbles, sinews, tissues, parasites, inclusions, structure/substance abnormalities, foreign bodies or the like.

27. Apparatus according to any of claims 24 to 26, **characterised in that** the at least one measuring and control means (31) of the inspection device (3) is designed for measuring at least one texture of the edible products as well as for generating a corresponding variable, the texture being recognisable by at least one meat area, at least one layer area, at least one bone area and/or a structure combining such areas.

28. Apparatus according to any of claims 25 to 27, **characterised in that** at least one control means (31, 32, 33, 34) of the inspection device (3) generates the control variable and/or control variables which are determined by at least one ratio, for control of at least one processing step according to at least one standard, which are determined by characteristics of the meat product or edible product (7) to be processed and/or processing parameters.

29. Apparatus according to any of claims 25 to 28, **characterised in that** at least one measuring and control means (31) of the inspection device (3) is designed so as to detect tolerance residues and hence in combination an area of the edible products (7) which is at least partially free from tolerance residues for control of the separating device (2) before the inspection device (3) with a view to minimisation of tolerance residues left behind.

30. Apparatus according to claim 29, **characterised in that** the combined measuring and control means (31) comprises an X-ray measuring means and an optical measuring means.

31. Apparatus according to any of claims 24 to 30, **characterised in that** the separating device (2) in front of the inspection device (3) is equipped with at least one separating tool that can be controlled by the inspection device (3), and **in that** the inspection device (3) is connected in a feedback circuit (34) to the separating device (2) to minimise the tolerance residues in accordance with a comparison of nominal and actual values.

32. Apparatus according to any of claims 24 to 31, **characterised in that** the separator means (4) behind the inspection device (3) has, for separating edible products (7) with and without the tolerance residues, the separator device (41) which extracts edible products (72) with residues from the inspected stream of edible product.

33. Apparatus according to claim 32, **characterised in that** the separator device (41) behind the inspection device (3) is arranged and designed for the separation of the tolerance residues in conjunction with at least one trimming device (42) which receives edible products (72) with residues which are separated by the separator device (41) and makes them available after trimming for return to the product stream of the apparatus (1).

34. Apparatus according to claim 33, **characterised in that** the separator/trimming device (42) is equipped with a means for automatically separating the tolerance residues.

35. Apparatus according to claim 34, **characterised in that** the automatic trimming device (42) is connected by a coupling circuit to the inspection device (3) in such a way that trimming takes place in accordance with at least one property of the tolerance residues, meat product and/or edible product (7) which is detected by the inspection device (3).

## Revendications

1. Procédé de traitement de produits carnés de type et de forme quelconque, à base de poisson, de volaille ou autre, qui sont transportés en nombre sur une chaîne de traitement (10) et qui contiennent des éléments d'au moins un type qui doivent être séparés, appelés éléments à séparer, lesquels éléments ne sont pas consommables ou doivent être exclus de la consommation, les éléments à séparer étant séparés des produits carnés et de cette façon les produits à consommer (7), qui sont produits pour la consommation, étant contrôlés par un dispositif d'inspection automatique (3) pour analyser les résidus restant lors de la séparation, ledit procédé comportant les étapes suivantes :
i) les éléments à séparer sont séparés des produits carnés de sorte que, pour un certain nombre de produits à consommer (7), on obtient et on tolère des résidus minimaux d'au moins un type comme résidus tolérés pour le traitement ultérieur des produits à consommer (7), notamment pour optimiser la production de produits à consommer,
ii) les résidus tolérés sont réglés de façon à ce que l'apparition de produits à consommer (7) présentant les résidus tolérés est définie et laissée, quant au traitement souhaitée, à une fréquence qui permet de fixer à l'avance le processus de séparation notamment en fonction de la dimension des produits, de paramètres de traitement, de pratiques opératoires lors de l'approvisionnement et/ou de l'espèce du produit,
iii) le dispositif d'inspection automatique (3) est réglé pour contrôler les résidus tolérés restant et réglés à l'étape ii),
iv) les produits à consommer (71, 72), sans résidus et avec résidus tolérés, délivrés par le dispositif d'inspection (3) sont séparés.

2. Procédé selon la revendication 1, **caractérisé en ce que** les produits à consommer (71) contrôlés et délivrés par le dispositif d'inspection automatique (3) et présentant les résidus tolérés sont conditionnés en séparant, avantageusement manuellement, les résidus tolérés.

3. Procédé selon la revendication 2, **caractérisé en ce que** les produits à consommer (73) conditionnés ainsi que les produits à consommer (71), contrôlés comme étant sans reste et délivrés par le dispositif d'inspection (3), sont amenés à une chaîne de traitement commune (10).

4. Procédé selon la revendication 3, **caractérisé en ce qu'**au niveau de la chaîne de traitement commune (10) il est effectué en plus une inspection manuelle destinée à contrôler des résidus tolérés et un conditionnement manuel destiné à séparer les résidus tolérés.

5. Procédé selon la revendication 3 ou 4, **caractérisé en ce que**, au bout de la chaîne de traitement commune, il est effectué une inspection finale manuelle ou automatique pour contrôler les résidus tolérés.

6. Procédé selon l'une des revendications 1 à 5, **caractérisé en ce que**, au moyen du dispositif d'inspection (3), on mesure au moins une texture représentant des résidus tolérés et éventuellement au moins une autre texture ou substance des produits à consommer et on détermine au moins une grandeur qui représente une information qualitative, quantitative et/ou locale sur la substance ou texture des produits à consommer (7) et on utilise cette information pour influer sur au moins un processus de séparation en amont du dispositif d'inspection (3) et le cas échéant sur au moins un autre processus de traitement (U, D) en amont et/ou en aval du dispositif d'inspection (3).

7. Procédé selon la revendication 6, **caractérisé en ce qu'**une ou plusieurs textures à inspecter respectivement un ou plusieurs endroits de texture à séparer sont définis par des résidus tolérés qui contiennent des os, des cartilages, des vaisseaux sanguins, de la graisse, de la peau, des membranes, des vessies, des tendons, des tissus, des parasites, des inclusions, des structures ou substances anormales, des corps étrangers ou analogues.

8. Procédé selon la revendication 6 ou 7, **caractérisé en ce qu'**une ou plusieurs textures à inspecter sont définies par au moins une région carnée, au moins une région de couche, au moins une région osseuse et/ou une structure mixte de ces régions.

9. Procédé selon l'une des revendications 6 à 8, **caractérisé en ce qu'**au moins une grandeur de comparaison et/ou une relation de grandeurs de comparaison sont prescrites pour influer sur au moins un processus de traitement (U, D) conformément à la ou les prescriptions.

10. Procédé selon la revendication 9, **caractérisé en ce que** la ou les grandeurs prescrites sont définies conformément à des caractéristiques du produit carné à traiter, du produit à consommer (7) et/ou en fonction de paramètres de traitement.

11. Procédé selon l'une des revendications 6 à 10, **caractérisé en ce que** au moins une grandeur représentant le degré de production de produits à consommer, ou rendement, est déterminée au moyen du dispositif d'inspection (3) et est utilisée pour optimiser la production en utilisant la grandeur représentant le degré de production pour commander le traitement en amont du dispositif d'inspection afin de modifier de façon optimale ladite grandeur.

12. Procédé selon la revendication 11, **caractérisé en ce que** les produits à traiter sont des produits à base de poisson dont la peau est retirée, et **en ce que**, au moyen du dispositif d'inspection automatique (3), on mesure la couche graisseuse ou argentée représentant le degré de production après le retrait de la peau et on détermine au moins une grandeur correspondante qui est utilisée pour régler le retrait de la peau notamment conformément à une grandeur prescrite afin d'optimiser productions de produits à consommer.

13. Procédé selon l'une des revendications 6 à 12, **caractérisé en ce que** au moins une grandeur d'information de texture est déterminée pour minimiser les résidus tolérés lors de la séparation des éléments à séparer des produits carnés.

14. Procédé selon la revendication 13, **caractérisé en ce que** l'on détermine au moins deux grandeurs d'information de texture qui sont mises en relation l'une avec l'autre pour minimiser les résidus tolérés, au moins une relation définie étant prescrite pour obtenir des résidus tolérés minimaux.

15. Procédé selon la revendication 14, **caractérisé en ce qu'**une grandeur d'information de texture représentant le résidu toléré et une grandeur d'information de texture définie par une région dépourvue au moins partiellement de résidus tolérés sont déterminées au niveau d'un produit à consommer (7, 71) présentant un résidu toléré minimal.

16. Procédé selon la revendication 15, **caractérisé en ce que** l'on définit, pour un type de produit, un pourcentage moyen caractéristique de résidus tolérés dans le produit et un pourcentage moyen sans résidus suivant une distribution prescrite pour un processus de séparation minimisant de façon optimale les résidus tolérés.

17. Procédé selon l'une des revendications 1 à 16, **caractérisé en ce que** les éléments à séparer des produits carnés sont séparés de ceux-ci au moyen d'un dispositif de séparation automatique (2), et **en ce que** les grandeurs déterminées et/ou mesurées par le dispositif d'inspection (3) sont utilisées dans un montage à rétroaction (32) pour commander le processus de séparation afin de minimiser les résidus tolérés.

18. Procédé selon l'une des revendications 6 à 17, **caractérisé en ce que** les produits à consommer (71, 72), avec ou sans résidus, délivrés par le dispositif d'inspection automatique (3) sont séparés automatiquement l'un de l'autre grâce au fait que le dispositif d'inspection (3) commande un dispositif de séparation (41) qui fait sortir des produits à consommer avec résidus tolérés d'un flot de produits à consommer inspectés.

19. Procédé selon la revendication 18, **caractérisé en ce que** les produits à consommer (7) inspectés sont délivrés par le dispositif d'inspection (3) en au moins une rangée et avantageusement en deux rangées parallèles.

20. Procédé selon la revendication 19, **caractérisé en ce que** les produits à consommer (72), avec résidus tolérés, qui ont été sortis sont conditionnés pour séparer les résidus tolérés, et les produits à consommer conditionnés (73) sont de nouveau amenés en au moins une rangée à la chaîne de production (10).

21. Procédé selon l'une des revendications 6 à 20, **caractérisé en ce que** les produits à consommer (71), présentant des résidus tolérés, sont conditionnés automatiquement pour séparer les résidus tolérés, et ce grâce au fait que le dispositif d'inspection (3) commande un dispositif de conditionnement (42).

22. Procédé selon l'une des revendications 1 à 21, **caractérisé en ce que** les moitiés avant ou cages thoraciques de volailles sont traitées et les filets séparés sont contrôlés à l'aide du dispositif d'inspection automatique (3), la partie formant la Furcula, qui reste sous forme de résidu toléré, et la partie formant la chaire pectorale, qui reste sous forme de résidu tolérés, étant mesurées et les grandeurs déterminées étant mises dans une relation prescrite afin d'optimiser le processus de séparation.

23. Procédé selon l'une des revendications 1 à 22, **caractérisé en ce que** la présence de résidus tolérés et de sang est mesurée dans la région anatomique du produit à consommer au moyen du dispositif d'inspection (3) afin de contrôler dans cette région une propriété du produit influencée par l'abattage et d'optimiser le travail d'abattage.

24. Dispositif (1) de traitement de produits carnés de type et de forme quelconque, à base de poisson, de volaille ou autre, destiné à mettre en oeuvre le procédé selon l'une des revendications 1 à 23, ledit dispositif comportant un dispositif de séparation (2) destiné à séparer des éléments, appelés éléments à séparer, qui ne sont pas consommables ou qui doivent être exclus de la consommation, un dispositif d'inspection automatique (3) disposé en aval du dispositif de séparation (2) et destiné à contrôler les produits à consommer (7) produit par la séparation pour analyser les résidus restants et un moyen (4) disposé en aval du dispositif d'inspection (3), comportant un dispositif de séparation (41) et destiné à séparer des produits à consommer (71, 72), avec et sans résidus, qui sont distingués en fonction des résultats d'une inspection effectuée par le dispositif d'inspection (3) et délivrés séparément l'un de l'autre, le dispositif de séparation (2) étant équipé d'au moins un outil de séparation et le dispositif d'inspection automatique (3) étant destiné à contrôler les produits à consommer (71, 72) avec résidus, **caractérisé en ce que** le dispositif d'inspection (3) comporte au moins un moyen de mesure et de commande (31) qui mesure, quant à la qualité, la quantité et/ou l'endroit de l'apparition, les résidus tolérés d'au moins un type qui sont réglés à une fréquence prescrite au moyen du procédé selon l'une des revendications 1 à 23 et qui restent de façon répartie pendant le cycle opératoire à la fréquence prescrite par l'au moins un outil de séparation pour un certain nombre de produits à consommer (7) en vue du traitement ultérieur des produits à consommer (7), notamment de l'optimisation de la production de produits à consommer, le dispositif d'inspection automatique (3) étant destiné à contrôler ces résidus tolérés de sorte que le moyen de mesure et de commande (31) génère au moins une grandeur, représentant le résultat de la mesure à une fréquence réglée, pour commander au moins un processus de séparation et de traitement en amont du dispositif d'inspection (3).

25. Dispositif selon la revendication 24, **caractérisé en ce que** l'au moins un moyen de mesure et de commande (31) génère au moins une grandeur, représentant le résultat de la mesure, pour commander au moins un autre processus de traitement en amont et/ou en aval du dispositif d'inspection (3).

26. Dispositif selon la revendication 24 ou 25, **caractérisé en ce que** l'au moins un moyen de mesure et de commande (31) du dispositif d'inspection (3) est destiné à mesurer au moins un endroit de substance ou de texture des produits à consommer qui sont produits par le résidu toléré ainsi qu'à générer une grandeur correspondante, au moins un endroit de substance ou texture étant reconnaissable par des résidus, notamment des os, des cartilages, des vaisseaux sanguins, de la graisse, de la peau, des membranes, des vessies, des tendons, des tissus, des parasites, des inclusions, des structures ou substances anormales, des corps étrangers ou analogues.

27. Dispositif selon l'une des revendications 24 à 26, **caractérisé en ce que** l'au moins un moyen de mesure et de commande (31) du dispositif d'inspection (3) est destiné à mesurer au moins une texture des produits à consommer ainsi qu'à générer une grandeur correspondante, la texture étant reconnaissable par au moins une région carnée, au moins une région de couche, au moins une région osseuse et/ou une structure mixte de ces régions.

28. Dispositif selon l'une des revendications 25 à 27, **caractérisé en ce que** au moins un moyen de commande (31, 32, 33, 34) du dispositif d'inspection (3) génère la grandeur de commande et/ou les grandeurs de commande, qui sont définies par au moins une relation, pour commander au moins un processus de traitement selon au moins une prescription, lesquelles grandeurs sont définies par des caractéristiques du produit carné à traiter, du produit à consommer (7) et du paramètre de traitement.

29. Dispositif selon l'une des revendications 25 à 28, **caractérisé en ce que** l'au moins un moyen de mesure et de commande (31) du dispositif d'inspection (3) est conformé de façon à contrôler les résidus tolérés et ainsi en combinaison au moins une région des produits à consommer (7) qui est partiellement dépourvue de résidus tolérés afin de commander le dispositif de séparation (2) en amont du dispositif d'inspection (3) en vue de minimiser les résidus tolérés restants.

30. Dispositif selon la revendication 29, **caractérisé en ce que** le moyen combiné de mesure et de commande (31) comporte un moyen de mesure à rayons X et un moyen de mesure optique.

31. Dispositif selon l'une des revendications 24 à 30, **caractérisé en ce que** le dispositif de séparation (2) en amont du dispositif d'inspection (3) est équipé d'au moins un outil de séparation pouvant être commandé par le dispositif d'inspection (3), et **en ce que** le dispositif d'inspection (3) est relié, dans un montage à rétroaction (34), au dispositif de séparation (2) en vue de minimiser les résidus tolérés conformément à une comparaison de valeurs consigne/valeurs réelles.

32. Dispositif selon l'une des revendications 24 à 31, **caractérisé en ce que** le moyen de séparation (4), disposé en aval du dispositif d'inspection (3) et destiné à séparer des produits à consommer (7) avec et sans résidus tolérés, comporte le dispositif de séparation (41) qui fait sortir des produits à consommer (72) avec résidus d'un flot de produits à consommer inspectés.

33. Dispositif selon la revendication 32, **caractérisé en ce que** le dispositif de séparation (41) est disposé en aval du dispositif d'inspection (3) et est destiné à séparer les résidus tolérés en liaison avec au moins un dispositif de conditionnement (42) qui reçoit du dispositif de séparation (41) des produits à consommer (72) qui possèdent des résidus et qui sont séparés et les prépare après le conditionnement pour les renvoyer dans le flot de produits du dispositif (1).

34. Dispositif selon la revendication 33, **caractérisé en ce que** le dispositif de séparation et de conditionnement (42) est doté d'un moyen de séparation automatique des résidus tolérés.

35. Dispositif selon la revendication 34, **caractérisé en ce que** le dispositif de conditionnement automatique (42) est relié au moyen d'un circuit de couplage au dispositif d'inspection (3) de sorte que le processus de séparation et de conditionnement est effectué conformément à au moins une propriété du résidu toléré, du produit carné et/ou du produit à consommer (7) qui est déterminée par le dispositif d'inspection (3).
